# EUROPEAN PATENT APPLICATION

(11) **EP 4 368 085 A1**
(43) Date of publication of application: **15.05.2024**
(21) Application number: 22838037.4
(22) Date of filing: 08.07.2022
(51) Int. Cl.: A47L 9/16, A47L 9/14, A47L 9/12, A47L 9/10, A47L 9/20, A47L 9/28, A47L 7/00, A61L 2/10

(54) **VACUUM CLEANER STATION**

(30) Priority: 09.07.2021 US 202163219881 P; 06.07.2022 KR 20220083343
(71) Applicant: LG Electronics Inc., Yeongdeungpo-gu Seoul 07336 (KR)
(72) Inventor: KIM, Hanshin, Seoul 08592 (KR); HYUN, Kietak, Seoul 08592 (KR); SONG, Hyunsup, Seoul 08592 (KR); HER, Jonguk, Seoul 08592 (KR)
(74) Representative: Vossius & Partner Patentanwälte Rechtsanwälte mbB
(86) International application number: PCT/KR2022/009912
(87) International publication number: WO 2023/282685

(57) **Abstract**

The present disclosure relates to a cleaner station which is coupled to a cleaner in order to collect dust within a dust bin of the cleaner. A user can use either the first collector formed in the form of a bin having the fixed-sized dust receiving space or the second collector in the form of a bag having the variably-sized dust receiving space in combination with the cleaner station. Therefore, there is an advantage that it is possible to satisfy various preferences of users who prefer different types of collectors.

## Description

### BACKGROUND FIELD

The present disclosure relates to a cleaner station which is coupled to a cleaner in order to collect dust within a dust bin of the cleaner.

### Description of the Related Art

In a stick vacuum cleaner (hereinafter referred to as cleaner), since a dust bin that stores the collected dust has a small capacity, it is inconvenient for a user to empty the dust bin every time.

Accordingly, a cleaner station configured to suck and collect dust from the dust bin by a suction force of the dust collection motor are increasingly used in order for the user not to manually remove the dust from the dust bin.

The cleaner station includes a housing, a dust collection motor disposed within the housing, and a dust bag-type collector that receives the collected dust. The cleaner station is configured to be coupled to the cleaner or the dust bin of the cleaner.

The cleaner station is convenient in that it can automatically empty the dust bin of the cleaner. Also, since the dust bag included in the cleaner station has a larger volume than that of the dust bin provided in most cleaners, the cleaner has an advantage that a cycle in which the dust should be processed becomes longer.

On the other hand, the dust bag-type collector has a simple structure and the entire dust bag can be discarded in a wastebasket without separately removing dust or cleaning the inside thereof. Therefore, the dust bag-type collector is advantageous in terms of convenience of users.

However, since the inside of the dust bag cannot be cleaned, the dust bag must be purchased and replaced periodically, so that some users may feel burdened with the cost.

With regard to this, U.S. Registered Patent Publication No. 10595692 is presented as a prior art document.

The prior art document discloses an embodiment in which an evacuation station that docks with a robotic cleaner has a bin-type collector.

Unlike the dust bag, since the "bin"-type collector has its inside that can be easily cleaned, there is an advantage that the collector can be used semi-permanently only the dust received therein is removed.

However, there may be users who feel inconvenient to remove the dust and clean the collector, which are tasks that must be separately performed.

As such, if the collector has any fixed one type, it is difficult to satisfy different preferences of users.

### PRIOR ART DOCUMENT

U.S. Registered Patent Publication No. 10595692

### SUMMARY

The present disclosure is designed to overcome the problems, and the purpose of the present disclosure is to provide a cleaner station configured to satisfy various preferences of users who prefer different types of collectors.

The technical problem to be overcome in this document is not limited to the above-mentioned technical problems. Other technical problems not mentioned can be clearly understood from those described below by a person having ordinary skill in the art.

One embodiment is a cleaner station including: a housing which is coupled to a dust bin of a cleaner; a dust collection motor which is disposed within the housing and generates a suction force sucking dust within the dust bin; a collector that is provided with a dust receiving space which collects the dust sucked from the inside of the dust bin by the dust collection motor; and a chamber which is disposed in the housing and forms a collector receiving space such that the collector is separably coupled to the chamber.

The collector that is coupled to the chamber includes a first collector which is formed in the form of a bin having the fixed-sized dust receiving space and includes a cyclone as a dust separation means and a second collector which includes the variably-sized dust receiving space and a breathable material-made dust bag as the dust separation means. The first and second collectors are compatible.

The chamber includes a chamber body which forms an exterior of the collector receiving space.

The chamber includes a rail portion which is disposed on an upper portion of the chamber body, allows at least a portion of the second collector to be fitted and coupled thereto, and supports the second collector such that a bottom surface of the chamber body is spaced apart from the second collector by a predetermined distance.

The collector receiving space has a cross-sectional area of a front end thereof larger than a cross-sectional area of a rear end thereof.

The front of the chamber body is opened or closed by a housing cover rotatably coupled to one side of the housing.

The rail portion includes a rail body which forms a space in which the second collector slides.

The rail portion includes an interference protrusion which is disposed at a first position that interferes with the housing cover in front of the rail body and moves to a second position that avoids interference with the housing cover by contact with the first collector or the second collector.

The rail portion includes a protrusion guide which is coupled to the interference protrusion and guides movement of the interference protrusion.

The chamber includes a filter mounting portion which is disposed in a lower portion of the chamber body and to which a pre-filter module that filters air which has passed through the collector is coupled in an attachable and detachable manner.

The chamber includes a filter detection protrusion of which one side is pressed by the pre-filter module and which is moved in a direction to avoid interference with the first collector.

The pre-filter module includes: a filter case; a filter which is received within the filter case; and a pressing protrusion which is formed to protrude from one side of the filter case.

The filter detection protrusion includes: a protrusion body; and a catching protrusion which is formed to protrude from an outer surface of the protrusion body and comes into contact with the pressing protrusion as the pre-filter module is coupled to the filter mounting portion.

The chamber includes a compression driver which is disposed in a lower portion of the chamber body and generates power for rotating a compression rotating portion that compresses the dust collected in the first collector.

The first collector includes a transmission gear which is coupled to the compression rotating portion and transmits the power from the compression driver to the compression rotating portion.

The compression driver includes a compression motor which is disposed outside the collector receiving space.

The compression driver includes a drive gear which is connected to a shaft of the compression motor outside the collector receiving space and is meshed with the transmission gear.

A gear passing hole which exposes at least a portion of the drive gear toward an inside of the collector receiving space is formed in the lower portion of the chamber body such that the drive gear and the transmission gear are meshed with each other.

The compression driver includes a sealing member disposed between the drive gear and the chamber body in order to seal the gear passing hole.

The cleaner station according to the embodiment of the present disclosure includes a controller which detects a change in a rotation direction of the compression motor.

The controller determines a type of the collector coupled to the chamber on the basis of whether the change in a rotation direction of the compression motor is detected within a predetermined period of time.

The chamber includes an air inlet which is provided in a top of the chamber body and has an open cross-section having a rearward-downward inclination.

The chamber includes a sterilization module mounting portion which is disposed to be spaced apart from the air inlet and is equipped with a sterilization module that is provided to irradiate ultraviolet light toward the collector receiving space.

The first collector includes a collector body which forms an exterior of the dust receiving space.

The first collector includes a body cover which covers an open top of the collector body.

A rib which is inserted into the rail portion is formed to protrude from the body cover.

A rearward and downward inclined surface is formed in some areas of a top surface of the body cover.

A dust inlet positioned to face the air inlet is formed in the inclined surface, on the basis of a state where the first collector is inserted into the chamber body.

The second collector includes a breathable material-made dust bag.

The second collector includes an outer plate which is coupled to an outside top of the dust bag, is fitted and coupled to the rail portion, is inserted into the collector receiving space in a sliding manner, and includes a dust inlet formed therein.

The second collector includes a support member which is formed to protrude from a bottom surface of the outer plate, has a lower portion coming into contact with one surface of the rail portion, and supports a state where the outer plate is lifted toward the air inlet.

### BRIEF DESCRIPTION OF THE DRAWINGS

FIG. 1 is a view for describing a cleaner according to an embodiment of the present disclosure;
FIG. 2 is a view of the cleaner of FIG. 1 viewed from another angle;
FIG. 3 is a view for describing a bottom surface of a dust bin of the cleaner according to the embodiment of the present disclosure;
FIG. 4 is a view for describing a cleaner system according to the embodiment of the present disclosure;
FIG. 5 is a view for describing a coupling portion of a cleaner station according to the embodiment of the present disclosure;
FIG. 6 is a view for describing a fixing unit of the cleaner station according to the embodiment of the present disclosure;
FIG. 7 shows a state where a door has closed a dust passing hole;
FIG. 8 shows a state where the door has opened the dust passing hole;
FIG. 9 is a view for describing a relationship between the cleaner and a cover opening unit according to the embodiment of the present disclosure;
FIG. 10 is a view for describing arrangement between components of the cleaner station according to the embodiment of the present disclosure;
FIG. 11 is a perspective view showing a chamber and a collector formed in a shape that can be coupled to the chamber, according to the embodiment of the present disclosure;
FIG. 12 is a cross-sectional view of the chamber as viewed from the side thereof, in accordance with the embodiment of the present disclosure;
FIG. 13 is an enlarged cross-sectional view of an upper side portion of the chamber according to the embodiment of the present disclosure;
FIG. 14 is a view of the chamber viewed from the front thereof, in accordance with the embodiment of the present disclosure;
FIG. 15 is a perspective view showing an internal structure of the chamber according to the embodiment of the present disclosure;
FIG. 16 is s cross-sectional view showing a compression driver, in accordance with the embodiment of the present disclosure;
FIG. 17 is an enlarged view of a part "A" of FIG. 15;
FIG. 18 is a cross-sectional view taken along line C-C' of FIG. 17;
FIG. 19 is a view describing the movement of an interference protrusion and thus a position relationship with the interference protrusion and a housing cover, in accordance with the embodiment of the present disclosure;
FIG. 20 is a view showing that a pre-filter module is divided into constituents and developed in accordance with the embodiment of the present disclosure;
FIG. 21 shows an enlarged part "B" of FIG. 15 and is a view for describing a movement of a filter detection protrusion depending on whether the pre-filter module is mounted or not;
FIG. 22 is a cross-sectional view taken along line D-D' of FIG. 21;
FIG. 23 is a perspective view of a first collector according to the embodiment of the present disclosure;
FIG. 24 is a view showing that components related to the first collector are divided and developed in accordance with the embodiment of the present disclosure;
FIG. 25 is a view showing a dust separation process of a cyclone in accordance with the embodiment of the present disclosure;
FIG. 26 is a cross-sectional view taken along line X-X' of FIG. 23;
FIG. 27 shows an enlarged drive gear and an enlarge transmission gear according to the embodiment of the present disclosure;
FIG. 28 is a perspective view of the drive gear as viewed from the bottom according to the embodiment of the present disclosure;
FIG. 29 is a perspective view showing a state where the first collector is inserted into a chamber body according to the embodiment of the present disclosure;
FIG. 30 is a view showing a flow path of air related to dust collection in the state where the first collector is inserted into the chamber body according to the embodiment of the present disclosure;
FIG. 31 is a view showing that components related to a second collector are separated and deployed according to the embodiment of the present disclosure;
FIG. 32 is a cross-sectional view of the second collector as viewed from the side thereof according to the embodiment of the present disclosure;
FIG. 33 is a perspective view showing a state where the second collector is inserted into the chamber body according to the embodiment of the present disclosure;
FIG. 34 is a view showing a flow path of air related to dust collection in the state where the second collector is inserted into the chamber body according to the embodiment of the present disclosure; and
FIG. 35 is a cross-sectional view showing an enlarged sealing member that seals a gear passing hole according to the embodiment of the present disclosure.

### DETAILED DESCRIPTION

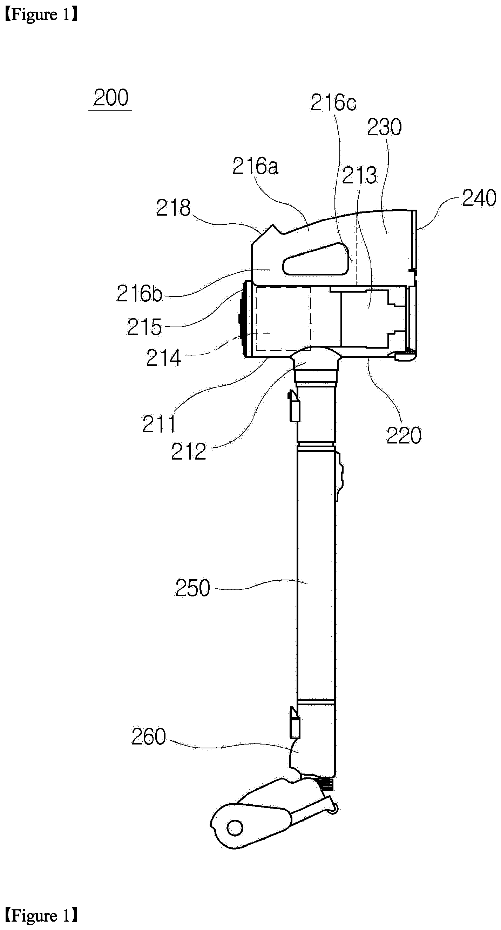

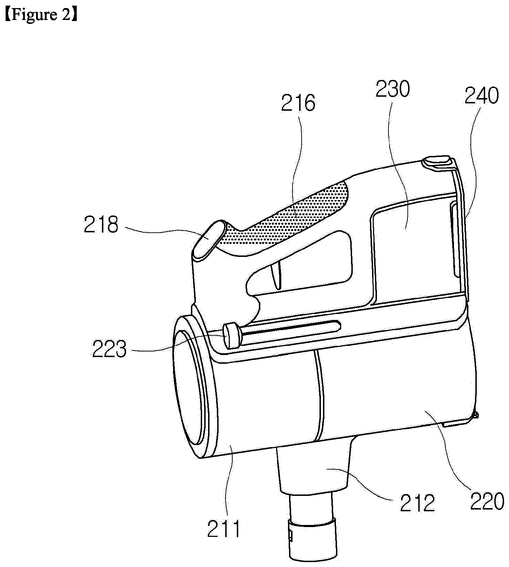

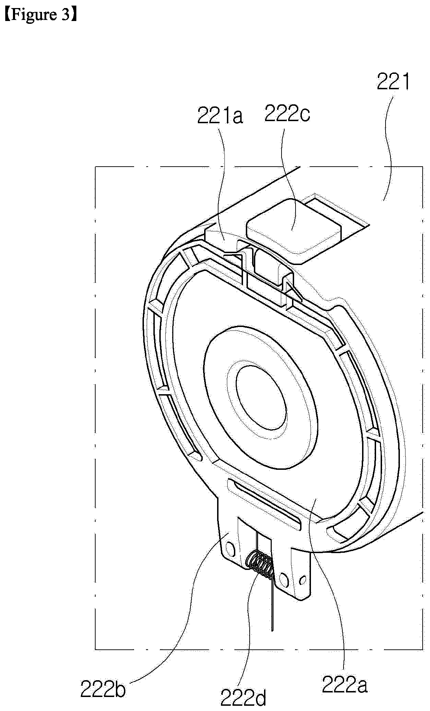

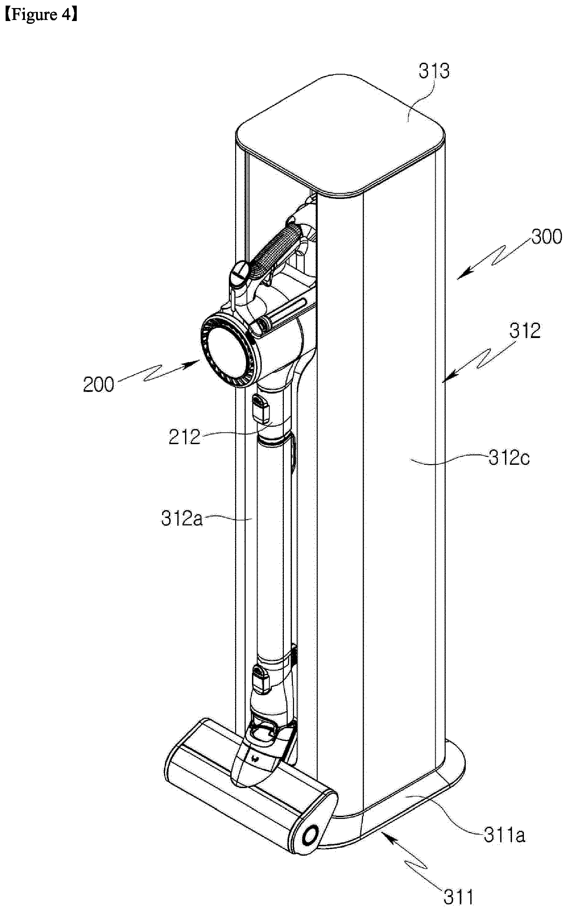

FIG. 1 is a view for describing a cleaner according to an embodiment of the present disclosure. FIG. 2 is a view of the cleaner of FIG. 1 viewed from another angle. FIG. 3 is a view for describing a bottom surface of a dust bin of the cleaner according to the embodiment of the present disclosure. FIG. 4 is a view for describing a cleaner system according to the embodiment of the present disclosure.

Referring to FIGS. 1 to 4, a cleaner system 3 according to the embodiment of the present disclosure may include a cleaner 200 and a cleaner station 300.

The cleaner system 3 may include the cleaner station 300. The cleaner 200 may be coupled to the cleaner station 300. The cleaner 200 may be coupled to the side of the cleaner station 300. The cleaner station 300 may remove dust of the dust bin 220 of the cleaner 200.

First, the structure of the cleaner 200 will be described with reference to FIGS. 1 to 3 as follows.

The cleaner 200 may mean a cleaner that is manually operated by a user. For example, the cleaner 200 may mean a handy-type cleaner or a stick-type cleaner.

The cleaner 200 may be mounted on the cleaner station 300. The cleaner 200 may be supported by the cleaner station 300. The cleaner 200 may be coupled to the cleaner station 300.

Meanwhile, in the embodiment of the present disclosure, the direction of the cleaner 200 can be defined based on when the bottom surface (lower surface) of a battery housing 230 and the dust bin 220 are placed on the ground.

Here, a front may refer to a direction in which a suction portion 212 is disposed with respect to a suction motor 214, and a rear may refer to a direction in which a handle 216 is disposed with respect to the suction motor 214. Also, a direction in which a component is disposed on the right side based on when the suction portion 212 is viewed from the suction motor 214 may be referred to as a right side, and a direction in which a component is disposed on the left side may be referred to a left side. In addition, in the embodiment of the present disclosure, upper and lower sides may be defined along a direction perpendicular to the ground based on when the bottom surface (lower surface) of a battery housing 230 and the dust bin 220 are placed on the ground.

The cleaner 200 may include a body 210. The body 210 may include a body housing 211, the suction portion 212, a dust separator 213, the suction motor 214, an air exhaust cover 215, the handle 216, and an operation unit 218.

The body housing 211 may form the exterior of the cleaner 200. The body housing 211 may provide a space for receiving the suction motor 214 and a filter (not shown) therein. The body housing 211 may be formed in a shape similar to a cylinder.

The suction portion 212 may protrude outward from the body housing 211. For example, the suction portion 212 may be formed in a cylindrical shape with an open interior. The suction portion 212 may be coupled to an extension tube 250. The suction portion 212 may provide a flow path (hereinafter, may be referred to as a "suction flow path") through which air including dust can flow.

The dust separator 213 may communicate with the suction portion 212. The dust separator 213 can separate dust sucked into the dust through the suction portion 212. The space within the dust separator 213 may communicate with a space within the dust bin 220.

For example, the dust separator 213 may include at least one cyclone capable of separating dust by cyclone flow. In addition, the space within the dust separator 213 may communicate with the suction flow path. Accordingly, the air and dust sucked through the suction portion 212 flow spirally along the inner peripheral surface of the dust separator 213. Therefore, cyclone flow may occur in an interior space of the dust separator 213.

The dust separator 213 communicates with the suction portion 212 and is configured by applying a principle in which a dust collector uses a centrifugal force, in order to separate the dust sucked into the body 210 through the suction portion 212.

The dust separator 213 may further include a secondary cyclone that again separates dust from the air exhausted from the cyclone. Here, the secondary cyclone may be positioned within the cyclone such that the size of the dust separator is minimized. The secondary cyclone may include a plurality of cyclone bodies arranged in parallel. The air exhausted from the cyclone may be divided into a plurality of cyclone bodies and passed through.

Here, the axis of the cyclone flow of the secondary cyclone may also extend in the up and down direction, and the axis of the cyclone flow of the cyclone and the axis of the cyclone flow of the secondary cyclone may be coaxial in the up and down direction. This may be collectively referred to as the axis of the cyclone flow of the dust separator 213.

The suction motor 214 may generate a suction force for sucking air. The suction motor 214 may be received within the body housing 211. The suction motor 214 may generate the suction force by rotation. For example, the suction motor 214 may be provided in a shape similar to a cylinder.

The air exhaust cover 215 may be disposed on one side of the body housing 211 in the axial direction. A filter for filtering air may be received in the air exhaust cover 215. For example, a HEPA filter may be received in the air exhaust cover 215.

An air outlet for exhausting air sucked by the suction force of the suction motor 214 may be formed in the air exhaust cover 215.

A flow guide may be disposed on the air exhaust cover 215. The flow guide may guide the flow of air that is exhausted through the air outlet.

The handle 216 may be gripped by the user. The handle 216 may be disposed at the rear of the suction motor 214. For example, the handle 216 may be formed in a shape similar to a cylinder. Alternatively, the handle 216 may be formed in a curved cylindrical shape. The handle 216 may be disposed at a predetermined angle with the body housing 211, the suction motor 214, or the dust separator 213.

The handle 216 may include a grip portion 216a formed in a column shape for allowing the user to hold, a first extension portion 216b which is connected to one longitudinal (axial) end of the grip portion 216a and extends toward the suction motor 214, and a second extension portion 216c which is connected to the other longitudinal (axial) end of the grip portion 216a and extends toward the dust bin 220.

The top surface of the handle 216 may form a partial exterior of the top surface of the cleaner 200. Through this, when the user grips the handle 216, it is possible to prevent one component of the cleaner 200 from coming into contact with the user's arm.

The first extension portion 216b may extend from the grip portion 216a toward the body housing 211 or the suction motor 214. At least a portion of the first extension portion 216b may extend in a horizontal direction.

The second extension portion 216c may extend from the grip portion toward the dust bin 220. At least a portion of the second extension portion 216c may extend in a horizontal direction.

The operation unit 218 may be disposed on the handle 216. The operation unit 218 may be disposed on an inclined surface formed in a top area of the handle 216. The user can input an operation command or a stop command of the cleaner 200 through the operation unit 218.

The cleaner 200 may include the dust bin 220. The dust bin 220 may communicate with the dust separator 213. The dust bin 220 may store dust separated by the dust separator 213.

The dust bin 220 may include a dust bin body 221, an exhaust cover 222, a dust bin compression lever 223, and a compressor (not shown).

The dust bin body 221 may provide a space for storing the dust separated by the dust separator 213. For example, the dust bin body 221 may be formed in a shape similar to a cylinder.

A lower surface (bottom surface) of the dust bin body 221 may be partially open. In addition, a lower surface extension portion 221a may be formed on the lower surface (bottom surface) of the dust bin body 221. The lower surface extension portion 221a may be formed to block a portion of the lower surface of the dust bin body 221.

The dust bin 220 may include the exhaust cover 222. The exhaust cover 222 may be disposed on the lower surface of the dust bin 220.

The exhaust cover 222 may be provided to open and close one longitudinal end of the dust bin body 221. Specifically, the exhaust cover 222 may selectively open and close the lower portion of the dust bin 220 that opens downward.

The exhaust cover 222 may include a cover body 222a and a hinge portion 222b. The cover body 222a may be formed to block a portion of the lower surface of the dust bin body 221. The cover body 222a may rotate downward with respect to the hinge portion 222b. The hinge portion 222b may be disposed adjacent to the battery housing 230. A torsion spring 222d may be provided on the hinge portion 222b. Accordingly, when the exhaust cover 222 is separated from the dust bin body 221, the cover body 222a can be supported in a state rotated at an angle greater than a predetermined angle from the dust bin body 221 about the hinge portion 222b, by the elastic force of the torsion spring 222d.

The exhaust cover 222 may be coupled to the dust bin 220 through hook coupling. Meanwhile, the exhaust cover 222 may be separated from the dust bin 220 through a coupling lever 222c. The coupling lever 222c may be disposed in front of the dust bin. Specifically, the coupling lever 222c may be disposed on the front outer surface of the dust bin 220. When an external force is applied, the coupling lever 222c may elastically deform a hook extending from the cover body 222a in such a way as to release the hook coupling between the cover body 222a and the dust bin body 221.

When the exhaust cover 222 is closed, the lower surface of the dust bin 220 may be blocked (sealed) by the exhaust cover 222 and the lower surface extension portion 221a.

The dust bin 220 may include the dust bin compression lever 223 (see FIG. 2). The dust bin compression lever 223 may be disposed outside the dust bin 220 or the dust separator 211. The dust bin compression lever 223 may be disposed to move up and down on the outside of the dust bin 220 or the dust separator 213. The dust bin compression lever 223 may be connected to the compressor (not shown). When the dust bin compression lever 223 moves downward by an external force, the compressor (not shown) may also move downward. Through this, it is possible to provide the user convenience. The compressor (not shown) and the dust bin compression lever 223 may return to their original positions by an elastic member (not shown). Specifically, when the external force applied to the dust bin compression lever 223 is removed, the elastic member may move the dust bin compression lever 223 and the compressor (not shown) upward.

A compressor (not shown) may be disposed within the dust bin body 221. The compressor may move in an interior space of the dust bin body 221. Specifically, the compressor may move up and down within the dust bin body 221. Through this, the compressor may compress the dust in the dust bin body 221 downward. In addition, when the exhaust cover 222 is separated from the dust bin body 221 and the lower portion of the dust bin 220 is opened, the compressor moves from the upper portion to the lower portion of the dust bin 220, and then can remove foreign substances such as residual dust in the dust bin 220. Through this, residual dust is not intended to remain in the dust bin 220, so that the suction force of the cleaner can be improved. Also, the residual dust is not intended to remain in the dust bin 220, so that it is possible to remove odors generated by the residue.

The cleaner 200 may include the battery housing 230. A battery 240 may be received in the battery housing 230. The battery housing 230 may be disposed below the handle 216. For example, the battery housing 230 may have a hexahedral shape with an open bottom. A rear surface of the battery housing 230 may be connected to the handle 216.

The battery housing 230 may include a receiving portion that is opened downward. The battery 240 may be attachable to and detachable through the receiving portion of the battery housing 220.

The cleaner 200 may include the battery 240.

For example, the battery 240 may be separably coupled to the cleaner 200. The battery 240 may be separably coupled to the battery housing 230. For example, the battery 240 may be inserted from below the battery housing 230 to the inside of the battery housing 230. Such a configuration can improve the portability of the cleaner 200.

Unlike this, the battery 240 may be integrally provided within the battery housing 230. Here, the bottom surface of the battery 240 is not exposed to the outside.

The battery 240 may supply power to the suction motor 214 of the cleaner 200. The battery 240 may be disposed below the handle 216. The battery 240 may be disposed at the rear of the dust bin 220.

According to the embodiment, when the battery 240 is coupled to the battery housing 230, the bottom surface of the battery 240 may be exposed to the outside. Since the battery 240 may be placed on the floor when the cleaner 200 is placed on the floor, the battery 240 can be directly separated from the battery housing 230. Also, since the bottom surface of the battery 240 is exposed to the outside and comes in direct contact with the external air of the battery 240, the cooling performance of the battery 240 can be improved.

Meanwhile, when the battery 240 is integrally fixed to the battery housing 230, the structure for attaching and detaching the battery 240 and the battery housing 230 can be reduced, so that the overall size of the cleaner 200 can be reduced and the weight of the cleaner 200 can be reduced.

The cleaner 200 may include the extension tube 250. The extension tube 250 may communicate with the cleaning module 260. The extension tube 250 may communicate with the body 210. The extension tube 250 may communicate with the suction part 214 of the body 210. The extension tube 250 may be formed in a long cylindrical shape.

The body 210 may be connected to the extension tube 250. The body 210 may be connected to a cleaning module 260 through the extension tube 250. The body 210 may generate a suction force by the suction motor 214 and may provide the suction force to the cleaning module 260 through the extension tube 250. External dust may be introduced into the body 210 through the cleaning module 260 and the extension tube 250.

The cleaner 200 may include the cleaning module 260. The cleaning module 260 may communicate with the extension tube 250. Accordingly, external air may be introduced into the body 210 of the cleaner 200 via the cleaning module 260 and the extension tube 250 by the suction force generated in the body 210 of the cleaner 200.

Dust in the dust bin 220 of the cleaner 200 may be collected by a collector 500 of the cleaner station 300 by gravity. At the same time, the dust in the dust bin 220 may be collected to the collector 500 of the cleaner station 300 by the suction force of a dust collection motor 391 disposed within the cleaner station 300. Through this, since the dust in the dust bin of the cleaner can be removed without a separate operation of the user, convenience for users can be provided. In addition, it is possible to remove the inconvenience for the user to empty the dust bin each time. Also, the dust can be prevented from scattering during the emptying of the dust bin.

The cleaner 200 may be coupled to a side of a housing 310. Specifically, the body 210 of the cleaner 200 may be mounted on a coupling portion 320. More specifically, the dust bin 220 and the battery housing 230 of the cleaner 200 may be coupled to a coupling surface 321 of the coupling portion 320. The outer peripheral surface of the dust bin body 221 may be coupled to a dust bin guide surface 322. The suction portion 212 may be coupled to a suction portion guide surface 326 of the coupling portion 320. With this configuration, the central axis of the dust bin 220 may be disposed in a direction parallel to the ground, and the extension tube 250 may be disposed along a direction perpendicular to the ground.

Hereinafter, the configuration of the cleaner station 300 according to the embodiment of the present disclosure will be described.

Referring to FIG. 4, the cleaner 200 may be coupled to the cleaner station 300. Specifically, the body of the cleaner 200 may be coupled to the side of the cleaner station 300. More specifically, the dust bin 220 of the cleaner 200 is coupled to the side of the cleaner station 300 through one side on which the exhaust cover 222 is disposed. Accordingly, when the exhaust cover 222 is opened, the dust of the dust bin 220 may be collected to the inside of the cleaner station 300 and removed.

The cleaner station 300 may include the housing 310. The housing 310 may form the exterior of the cleaner station 300. Specifically, the housing 310 may be formed in a column shape including at least one outer wall surface. For example, the housing 310 may be formed in a shape similar to a quadrangular column.

The housing 310 may have a space formed therein for receiving the collector 500 and a dust suction module 390.

The housing 310 may include a bottom surface 311, an outer wall surface 312, and a top surface 313.

The bottom surface 311 may support the lower portion in the gravity direction of the dust suction module 390. That is, the bottom surface 311 may support the lower portion of the dust collection motor 391 of the dust suction module 390.

Here, the bottom surface 311 may be placed toward the ground. The bottom surface 311 can be disposed to be inclined at a predetermined angle to the ground as well as can be placed parallel to the ground. With such a configuration, the bottom surface is able to stably support the dust collection motor 391, and is able to balance the overall weight even when the cleaner 200 is coupled.

Meanwhile, according to the embodiment, the bottom surface 311 may further include a ground support portion 311a that increases an area in contact with the ground in order to prevent the cleaner station 300 from falling over and to maintain the balance. For example, the ground support portion 311a may be formed in the form of a plate extending from the bottom surface 311, or may be formed by that one or more frames protrude and extend from the bottom surface 311 in the direction of the ground.

The outer wall surface 312 may mean a surface formed in the direction of gravity, and may mean a surface connected to the bottom surface 311. For example, the outer wall surface 312 may mean a surface connected perpendicular to the bottom surface 311. In another embodiment, it is possible that the outer wall surface 312 is disposed to be inclined at a predetermined angle with the bottom surface 311.

The outer wall surface 312 may include at least one surface. For example, the outer wall surface 312 may include a first outer wall surface 312a, a second outer wall surface 312b, a third outer wall surface 312c, and a fourth outer wall surface 312d.

Here, in this embodiment, the first outer wall surface 312a may be disposed on the front of the cleaner station 300. Here, the front may refer to a surface to which the cleaner 200 is exposed, in the state where the cleaner 200 is coupled to the cleaner station 300. Accordingly, the first outer wall surface 312a may form the exterior of the front of the cleaner station 300.

Meanwhile, the direction is defined as follows for understanding the present embodiment. In the present embodiment, the direction may be defined in the state where the cleaner 200 is coupled to the cleaner station 300.

When the cleaner 200 is coupled to the cleaner station 300, a direction in which the cleaner 200 is exposed to the outside of the cleaner station 300 may be referred to as a front.

From another point of view, when the cleaner 200 is coupled to the cleaner station 300, the direction in which the suction motor 214 of the cleaner 200 is disposed may be referred to as a front. Also, a direction opposite to the direction in which the suction motor 214 is disposed in the cleaner station 300 may be referred to as a rear.

Also, a surface facing the front with respect to an interior space of the housing 310 may be referred to as a rear surface of the cleaner station 300. Accordingly, the rear surface may refer to a direction in which the second outer wall surface 312b is formed.

Also, a surface on the left when the front is viewed with reference to the interior space of the housing 310 may be referred to as a left surface, and a surface on the right may be referred to as a right surface. Accordingly, the left surface may mean a direction in which the third outer wall surface 312c is formed, and the right surface may mean a direction in which the fourth outer wall surface 312d is formed.

The first outer wall surface 312a may be formed not only in a flat shape, but also in a curved shape as a whole, or may be formed to include a partial curved surface.

The coupling portion 320 may be disposed on the first outer wall surface 312a. With this configuration, the cleaner 200 may be coupled to the cleaner station 300 and may be supported by the cleaner station 300. A detailed configuration of the coupling portion 320 will be described later.

Meanwhile, a structure for mounting various types of cleaning modules 260 which are used in the cleaner 200 can be added to the first outer wall surface 312a.

In this embodiment, the second outer wall surface 312b may face the first outer wall surface 312a. That is, the second outer wall surface 312b may be disposed on the rear surface of the cleaner station 300. The second outer wall surface 312b may form the exterior of the rear surface of the cleaner station 300.

In this embodiment, the third outer wall surface 312c and the fourth outer wall surface 312d may refer to surfaces connecting the first outer wall surface 312a and the second outer wall surface 312b. Here, the third outer wall surface 312c may be disposed on the left surface of the cleaner station 300, and the fourth outer wall surface 312d may be disposed on the right surface of the cleaner station 300. Unlike this, it is also possible that the third outer wall surface 312c may be disposed on the right surface of the cleaner station 300, and the fourth outer wall surface 312d may be disposed on the left surface of the cleaner station 300.

The third outer wall surface 312c or the fourth outer wall surface 312d may be formed not only in a flat shape, but also in a curved shape as a whole, or may be formed to include a partial curved surface.

Meanwhile, a structure for mounting various types of cleaning modules 260 which are used in the cleaner 200 can be added to the third outer wall surface 312c or the fourth outer wall surface 312d.

The top surface 313 may form the upper exterior of the cleaner station. That is, the top surface 313 may mean a surface that is disposed at the upper side in the direction of gravity in the cleaner station and is exposed to the outside.

For reference, in the present embodiment, the upper and lower sides may be defined respectively along the direction of gravity (direction perpendicular to the ground) in a state where the cleaner station 300 is installed on the ground.

Here, the top surface 313 can be disposed to be inclined at a predetermined angle to the ground as well as can be placed parallel to the ground.

A display unit 530 may be disposed on the top surface 313. For example, the display unit 530 may display the state of the cleaner station 300 and the state of the cleaner 200, and may additionally display information on a cleaning progress status and on a map of a cleaning area, etc.

Meanwhile, according to the embodiment, the top surface 313 may be provided to be separable from the outer wall surface 312. Here, when the top surface 313 is separated, the battery separated from the cleaner 200 can be received in the interior space surrounded by the outer wall surface 312, and a terminal (not shown) for charging the separated battery is provided in the interior space.

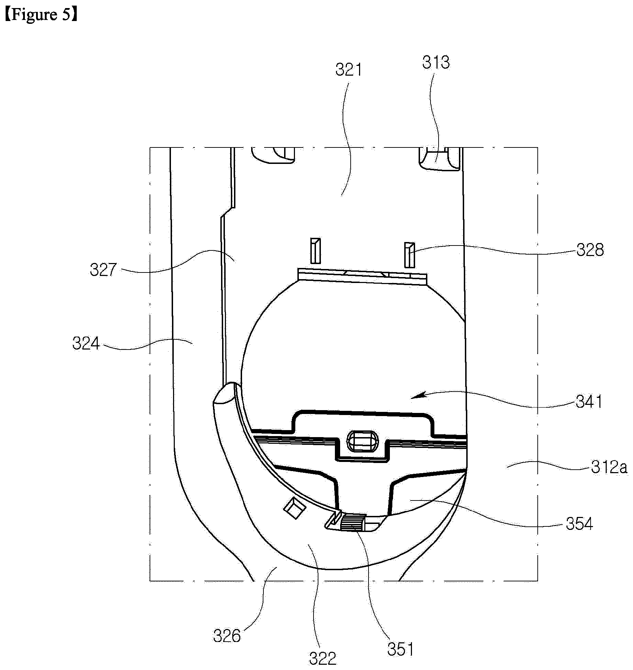

FIG. 5 is a view for describing the coupling portion of the cleaner station according to the embodiment of the present disclosure.

Referring to FIG. 5, the cleaner station 300 may include the coupling portion 320 for coupling the cleaner 200. Specifically, the coupling portion 320 may be disposed on the first outer wall surface 312a, and the dust bin 220 of the cleaner 200 may be coupled to the coupling portion 320. The body 210 and the battery housing 230 of the cleaner 200 as well as the dust bin 220 may also be coupled to the coupling portion 320.

The coupling portion 320 may include the coupling surface 321. The coupling surface 321 may be disposed on a side surface of the housing 310. For example, the coupling surface 321 may mean a surface formed in a shape of a groove concave toward the inside of the cleaner station 300 from the first outer wall surface 312a. That is, the coupling surface 321 may be formed to have a step difference with respect to the first outer wall surface 312a.

The cleaner 200 may be coupled to the coupling surface 321. For example, the coupling surface 321 may be in contact with the low surfaces of the dust bin 220 and the battery housing 230 of the cleaner 200. Here, the lower surface may mean a surface facing the ground when the user uses the cleaner 200 or puts it on the ground.

For example, an angle between the coupling surface 321 and the ground may be a right angle. Through this, when the cleaner 200 is coupled to the coupling surface 321, the space of the cleaner station 300 can be minimized.

For another example, the coupling surface 321 may be disposed to be inclined at a predetermined angle to the ground. Through this, when the cleaner 200 is coupled to the coupling surface 321, the cleaner station 300 may be stably supported.

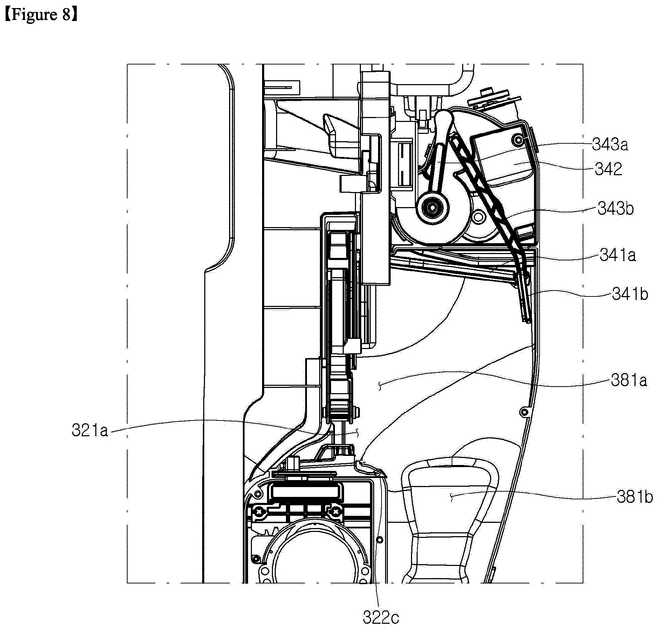

A dust passing hole 321a may be formed in the coupling surface 321 in order to allow external air of the housing 310 to flow into the coupling surface 321. The dust passing hole 321a may be formed in a hole shape corresponding to the shape of the dust bin 220 such that the dust of the dust bin 220 flows into the collector 500. The dust passing hole 321a may be formed to correspond to the shape of the exhaust cover 222 of the dust bin 220. The dust passing hole 321a may be formed to communicate with a flow path 380 to be described later (see FIG. 8).

The coupling portion 320 may include a dust bin guide surface 322. The dust bin guide surface 322 may be disposed on the first outer wall surface 312a. The dust bin guide surface 322 may be connected to the first outer wall surface 312a. Also, the dust bin guide surface 322 may be connected to the coupling surface 321.

The dust bin guide surface 322 may be formed to have a shape corresponding to the outer surface of the dust bin 220. The front outer surface of the dust bin 220 may be coupled to the dust bin guide surface 322. Through this, it is possible to provide convenience that allows the cleaner 200 to be coupled to the coupling surface 321.

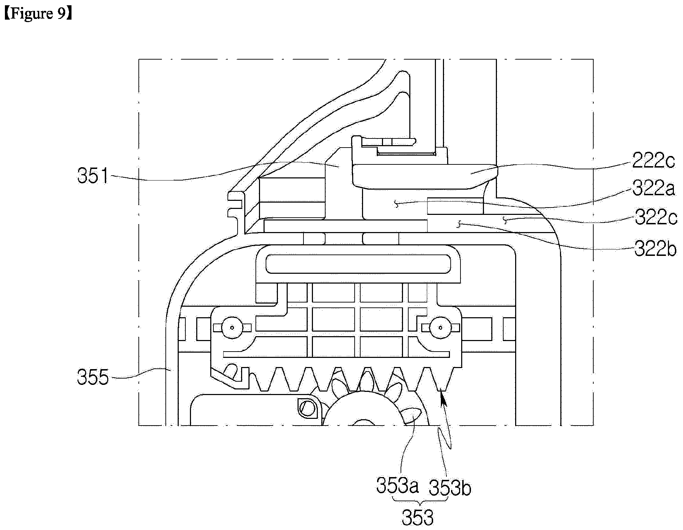

Meanwhile, a protrusion moving hole 322a may be formed on the dust bin guide surface 322, and a push protrusion 351 to be described later may move in a straight line along the protrusion moving hole 322a (see FIG. 9). In addition, a gear box 355 in which a gear of a cover opening unit 350 to be described later, etc., is received may be provided below the dust bin guide surface 322 in the direction of gravity. Here, a guide space 322b through which the push protrusion 351 can move may be formed between the dust bin guide surface 322, the lower surface, and the top surface of the gear box 355. Also, the guide space 322b may communicate with a first flow path 381 through a bypass hole 322c. That is, the protrusion moving hole 322a, the guide space 322b, the bypass hole 322c, and the first flow path 381 may form one flow path. With this configuration, when the dust collection motor 391 is operated in the state where the dust bin 220 is coupled to the coupling portion 320, the dust, etc., remaining in the dust bin 220 and in the dust bin guide surface 322 can be sucked through the flow path.

The coupling portion 320 may include a guide protrusion 323. The guide protrusion 323 may be disposed on the coupling surface 321. The guide protrusion 323 may protrude upward from the coupling surface 321. Two guide protrusions 323 may be disposed to be spaced apart from each other. A distance between the two guide protrusions 323 spaced apart from each other may correspond to the width of the battery housing 230 of the cleaner 200. Through this, it is possible to provide convenience that allows the cleaner 200 to be coupled to the coupling surface 321.

The coupling portion 320 may include a coupling portion sidewall 324. The coupling portion sidewall 324 may mean wall surfaces disposed on both sides of the coupling surface 321 and may be connected perpendicular to the coupling surface 321. The coupling portion sidewall 324 may be connected to the first outer wall surface 312a. In addition, the coupling portion sidewall 324 may form a surface connected to the dust bin guide surface 322. Through this, the cleaner 200 can be stably received.

The coupling portion 320 may include a coupling sensor. The coupling sensor may detect whether the cleaner 200 is coupled to the coupling portion 320.

The coupling sensor may include a contact sensor. For example, the coupling sensor may include a micro switch. Here, the coupling sensor may be disposed on the guide protrusion 323. Therefore, when the battery housing 230 or the battery 240 of the cleaner 200 is coupled between a pair of guide protrusions 323, the coupling sensor is brought into contact, and the coupling sensor can detect that the cleaner 200 is coupled.

On the other hand, the coupling sensor may include a non-contact sensor. For example, the coupling sensor may include an infrared sensor (IR sensor). Here, the coupling sensor may be disposed on the coupling portion sidewall 324. Therefore, when the dust bin 220 or the body 210 of the cleaner 200 passes through the coupling portion sidewall 324 and reaches the coupling surface 321, the coupling sensor detects the presence of the dust bin 220 or the body 210.

In the state where the cleaner 200 is coupled to the cleaner station 300, the coupling sensor may face the dust bin 220 or the battery housing 230 of the cleaner 200.

The coupling sensor may be a means for determining not only whether power is applied to the battery 240 of the cleaner 200 but also whether the cleaner 200 is coupled.

The coupling portion 320 may include the suction portion guide surface 326. The suction portion guide surface 326 may be disposed on the first outer wall surface 312a. The suction portion guide surface 326 may be connected to the dust bin guide surface 322. The suction portion 212 may be coupled to the suction portion guide surface 326. The shape of the suction portion guide surface 326 may be formed to correspond to the shape of the suction portion 212.

The coupling portion 320 may further include a fixing member entry and exit hole 327. The fixing member entry and exit hole 327 may be formed in the form of a long hole along the coupling portion sidewall 324 such that a fixing member 331 can enter and exit.

With this configuration, when the user couples the cleaner 200 to the coupling portion 320 of the cleaner station 300, the body 210 of the cleaner 200 can be stably disposed on the coupling portion 320 by the dust bin guide surface 322, the guide protrusion 323, and the suction portion guide surface 326. Through this, it is possible to provide convenience that allows the dust bin 220 and the battery housing 230 of the cleaner 200 to be coupled to the coupling surface 321.

Meanwhile, the cleaner station 300 may further include a charging terminal 328. The charging terminal 328 may be disposed on the coupling portion 320. The charging terminal 328 may be electrically connected to the cleaner 200 coupled to the coupling portion 320. The charging terminal 328 may supply power to the battery of the cleaner 200 coupled to the coupling portion 320.

Also, the cleaner station 300 may further include a side door. The side door may be disposed in the housing 310. The side door may selectively expose the collector 500 to the outside. Through this, the user is allowed to easily remove the collector 500 from the cleaner station 300.

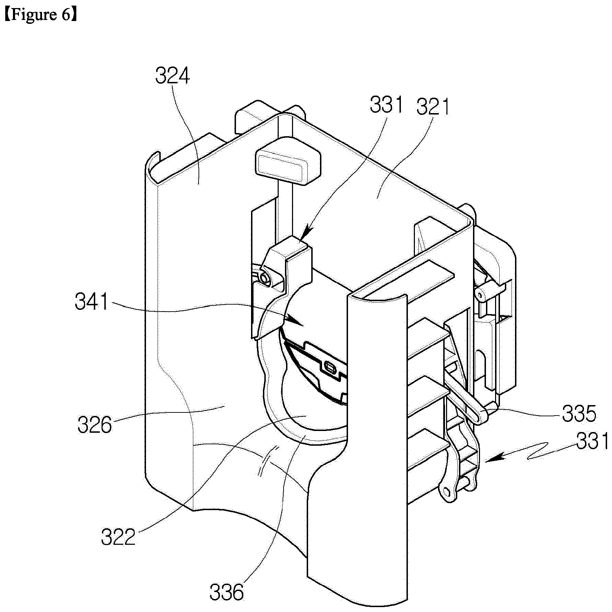

FIG. 6 is a view for describing a fixing unit of the cleaner station according to the embodiment of the present disclosure.

Referring to FIG. 6, the cleaner station 300 of the present disclosure may include a fixing unit 330. The fixing unit 330 may be disposed on the coupling portion sidewall 324. Also, the fixing unit 330 may be disposed on the back surface of the coupling surface 321. The fixing unit 330 may fix the cleaner 200 coupled to the coupling surface 321. Specifically, the fixing unit 330 may fix the dust bin 220 and the battery housing 230 of the cleaner 200 coupled to the coupling surface 321.

The fixing unit 330 may include a fixing member 331 for fixing the dust bin 220 and the battery housing 230 of the cleaner 200 and may include a fixing portion motor 580 for driving the fixing member 331. In addition, the fixing unit 330 may further include a fixing portion link 335 for transmitting the power of the fixing portion motor 580 to the fixing member 331.

The fixing member 331 may be disposed on the coupling portion sidewall 324, and may be provided to be able to perform a reciprocating movement on the coupling portion sidewall 324 so as to fix the dust bin 220. Specifically, the fixing member 331 may be received within the fixing member entry and exit hole 327.

The fixing member 331 may be disposed on both sides of the coupling portion 320 respectively. For example, two fixing members 331 may be disposed in pairs symmetrically with respect to the coupling surface 321.

The fixing portion motor 580 may provide power to move the fixing member 331.

The fixing portion link 335 may convert the rotational force of the fixing portion motor 580 into a reciprocating movement of the fixing member 331.

A fixed sealer 336 may be disposed on the dust bin guide surface 322 to seal the dust bin 220 when the cleaner 200 is coupled thereto. With this configuration, when the dust bin 220 of the cleaner 200 is coupled, the fixed sealer 336 can be pressed by the weight of the cleaner 200, and the dust bin 220 and the dust bin guide surface 322 are sealed. can be

The fixed sealer 336 may be disposed on an imaginary extension line of the fixing member 331. With such a configuration, when the fixing portion motor 580 is operated and the fixing member 331 presses the dust bin 220, the perimeter of the dust bin 220 at the same height can be sealed.

According to the embodiment, the fixed sealer 336 may be disposed on the dust bin guide surface 322 in the form of a bent line corresponding to the arrangement of the cover opening unit 350 to be described later.

Accordingly, when the body 210 of the cleaner 200 is disposed on the coupling portion 320, the fixing unit 330 may fix the body 210 of the cleaner 200. Specifically, when the coupling sensor 325 detects that the body 210 of the cleaner 200 is coupled to the coupling portion 320 of the cleaner station 300, the fixing portion motor 580 moves the fixing member 331, and then fixes the body 210 of the cleaner 200.

Through this, residual dust is not intended to remain in the dust bin, so that the suction force of the cleaner can be improved. Also, the residual dust is not intended to remain in the dust bin, so that it is possible to remove odors generated by the residue.

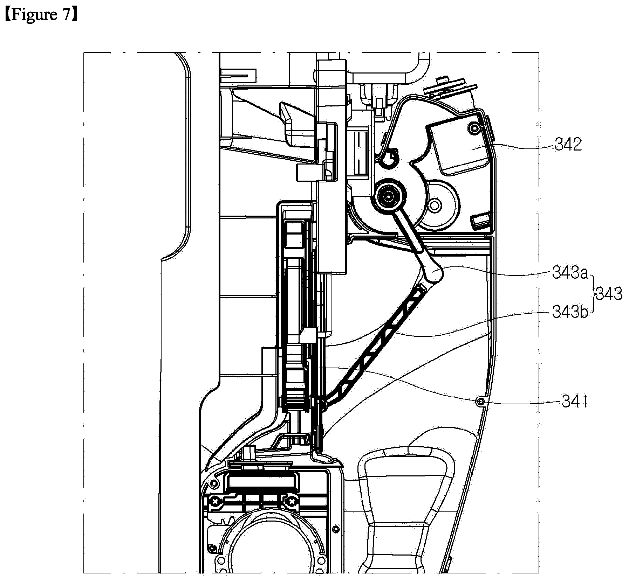

FIGS. 7 and 8 are views for describing a relationship between the cleaner and a door unit in the cleaner station according to the embodiment of the present disclosure. FIG. 7 shows a state where a door has closed a dust passing hole. FIG. 8 shows a state where the door has opened the dust passing hole.

Referring to FIGS. 7 and 8, the cleaner station 300 of the present disclosure may include a door unit 340. The door unit 340 may be configured to open and close the dust passing hole 321a.

The door unit 340 may include a door 341, a door motor 342, and a door arm 343.

The door 341 may be hinged to the coupling surface 321 and may open and close the dust passing hole 321a. The door 341 may include a door body 341a.

The door body 341a may be formed in a shape that can block the dust passing hole 321a. For example, the door body 341a may be formed in a shape similar to a disk.

Based on the state where the door body 341a blocks the dust passing hole 321a, the hinge portion is disposed over the door body 341a, and the arm coupling portion 341b is disposed below the door body 341a.

The door body 341a may be formed in a shape that can seal the dust passing hole 321a. For example, the outer surface of the door body 341a exposed to the outside of the cleaner station 300 is formed to have a diameter corresponding to the diameter of the dust passing hole 321a, and the inner surface of the door body 341a disposed within the cleaner station 300 is formed to have a diameter larger than that of the dust passing hole 321a. In addition, a step difference may be formed between the outer surface and the inner surface. Meanwhile, at least one reinforcing rib for connecting the hinge portion and the arm coupling portion 341b and for strengthening the supporting force of the door body 341a may be formed to protrude from the inner surface.

The hinge portion may be a means for hinge-coupling the door 341 to the coupling surface 321. The hinge portion may be disposed on the upper end of the door body 341a and may be coupled to the coupling surface 321.

The arm coupling portion 341b may be a means to which the door arm 343 is rotatably coupled. The arm coupling portion 341b may be disposed below the door body 341a and may be rotatably coupled to the door body 341a. The door arm 343 may be rotatably coupled to the arm coupling portion 341b.

With this configuration, when the door arm 343 pulls the door body 341a in a state where the door 341 closes the dust passing hole 321a, the door body 341a moves rotationally toward the inside of the cleaner station 300 about the hinge portion and the dust passing hole 321a may be opened. On the other hand, in the state where the dust passing hole 321a is opened, when the door arm 343 pushes the door body 341a, the door body 341a moves rotationally toward the outside of the cleaner station 300 about the hinge portion, and the dust passing hole 321a may be blocked.

Meanwhile, in a state where the cleaner 200 is coupled to the cleaner station 300 and the exhaust cover 215 is separated from the dust bin body 221, the door 341 may contact the exhaust cover 215. Also, according to the rotation of the door 341, the exhaust cover 215 may rotate in conjunction with the door 341.

The door motor 342 may provide power to rotate the door 341. Specifically, the door motor 342 may rotate the door arm 343 in a forward or reverse direction. Here, the forward direction may mean a direction in which the door arm 343 pulls the door 341. Accordingly, when the door arm 343 rotates in the forward direction, the dust passing hole 321a may be opened. Also, the reverse direction may mean a direction in which the door arm 343 pushes the door 341. Accordingly, when the door arm 343 rotates in the reverse direction, the dust passing hole 321a may be at least partially closed. The forward direction may be opposite to the reverse direction.

The door arm 343 may connect the door 341 and the door motor 342 and may open and close the door 341 by using the power generated from the door motor 342.

For example, the door arm 343 may include a first door arm 343a and a second door arm 343b. One end of the first door arm 343a may be coupled to the door motor 342. The first door arm 343a may be rotated by the power of the door motor 342. The other end of the first door arm 343a may be rotatably coupled to the second door arm 343b. The first door arm 343a may transmit the force transmitted from the door motor 342 to the second door arm 343b. One end of the second door arm 343b may be coupled to the first door arm 343a. The other end of the second door arm 343b may be coupled to the door 341. The second door arm 343b may open or close the dust passing hole 321a by pushing or pulling the door 341.

When the exhaust cover 222 of the cleaner 200 is opened, the door unit 340 may be opened together. Also, when the door unit 340 is closed, the exhaust cover 222 of the cleaner 200 may be closed in conjunction with the door unit.

When the dust in the dust bin 220 of the cleaner 200 is removed, the door motor 342 rotates the door 341 to couple the exhaust cover 222 to the dust bin body 221. Specifically, the door 341 is rotated about the hinge portion 341b by that the door motor 342 rotates the door 341. The door 141 rotating about the hinge portion 341b may push the exhaust cover 222 toward the dust bin body 221.

FIG. 9 is a view for describing a relationship between the cleaner and the cover opening unit according to the embodiment of the present disclosure.

Referring to FIG. 9, the cleaner station 300 of the present disclosure may include the cover opening unit 350. The cover opening unit 350 may be disposed on the coupling portion 320 and may open the exhaust cover 222 of the cleaner 200.

The cover opening unit 350 may include the push protrusion 351, a cover opening motor 352, a cover opening gear 353, and the gear box 355.

The push protrusion 351 may move to press the coupling lever 222c when the cleaner 200 is coupled.

The push protrusion 351 may be disposed on the dust bin guide surface 322. Specifically, the protrusion moving hole may be formed on the dust bin guide surface 322, and the push protrusion 351 may pass through the protrusion moving hole and may be exposed to the outside.

When the cleaner 200 is coupled, the push protrusion 351 may be disposed at a position which allows the push protrusion 351 to press the coupling lever 222c. That is, the coupling lever 222c may be disposed on the protrusion moving hole. In addition, the coupling lever 222c may be disposed on the moving region of the push protrusion 351.

The push protrusion 351 may perform a linear reciprocating motion in order to press the coupling lever 222c. Specifically, the push protrusion 351 may be coupled to the gear box 355 and may be guided in a linear motion. The push protrusion 351 may be coupled to the cover opening gear 353 and may move together by the movement of the cover opening gear 353.

The cover opening motor 352 may provide power to move the push protrusion 351. Specifically, the cover opening motor 352 may rotate a motor shaft in a forward or reverse direction. Here, the forward direction may mean a direction in which the push protrusion 351 presses the coupling lever 222c. Also, the reverse direction may mean a direction in which the push protrusion 351 pressing the coupling lever 222c is returned to its original position. The forward direction may be opposite to the reverse direction.

The cover opening gear 353 is coupled to the cover opening motor 352 and may move the push protrusion 351 by using the power of the cover opening motor 352. Specifically, the cover opening gear 353 may be receive within the gear box 355. A drive gear 353a of the cover opening gear 353 may be coupled to the motor shaft of the cover opening motor 352 and may receive power. A driven gear 353b of the cover opening gear 353 may be coupled to the push protrusion 351 and may move the push protrusion 351. For example, the driven gear 353b may be provided in the form of a rack gear, may mesh with the drive gear 353a, and may receive the power from the drive gear 353a.

Here, the exhaust cover 222 may be provided with the torsion spring 222d. The exhaust cover 222 may be rotated at a predetermined angle or more by the elastic force of the torsion spring 222d and may be supported at a position where the exhaust cover 222 has been rotated. Accordingly, the exhaust cover 222 may be opened and may communicate the dust passing hole 321a and the inside of the dust bin 220.

The gear box 355 may be provided within the housing 310 and may be disposed below the coupling portion 320 in the direction of gravity. The cover opening gear 353 may be received within the gear box.

According to the present disclosure, the cover opening unit 350 allows the user to open the dust bin 220 without separately opening the exhaust cover 222 of the cleaner, thereby improving convenience.

In addition, since the exhaust cover 222 is opened while the cleaner 200 is coupled to the cleaner station 300, it is possible to prevent the dust from scattering.

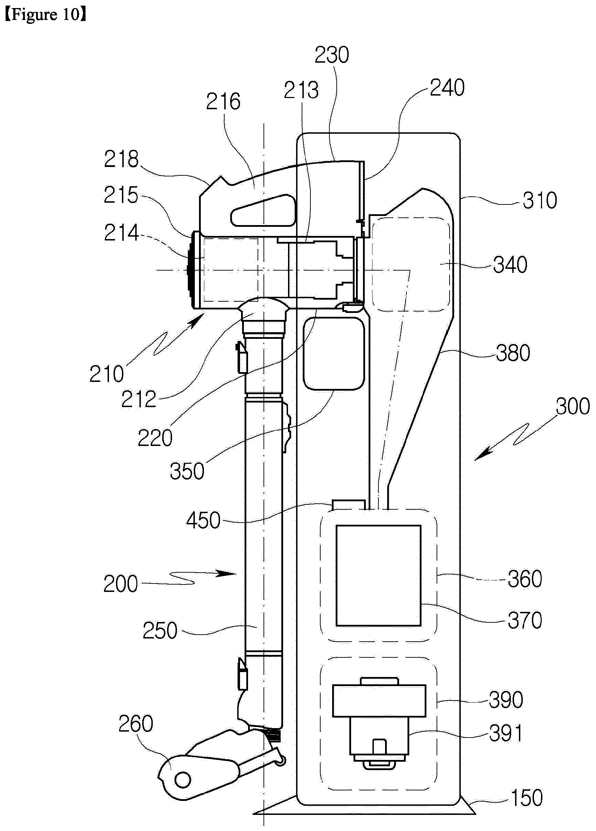

FIG. 10 is a view for describing arrangement between components of the cleaner station according to the embodiment of the present disclosure.

Referring to FIG. 10, the above-described cover opening unit 350 is disposed below the coupling portion 320, and the flow path 380 is disposed behind the coupling portion 320 and the cover opening unit 350.

Hereinafter, a flow path extending from the coupling portion 320 to the collector 500 is referred to as the first flow path 381. The collector 500 collecting the dust is disposed to be connected to the lower end of the first flow path 381.

The cleaner station 300 may further include a chamber 360.

The chamber 360 is disposed in the housing 310 and has a collector receiving space 360a formed therein for receiving the collector 500.

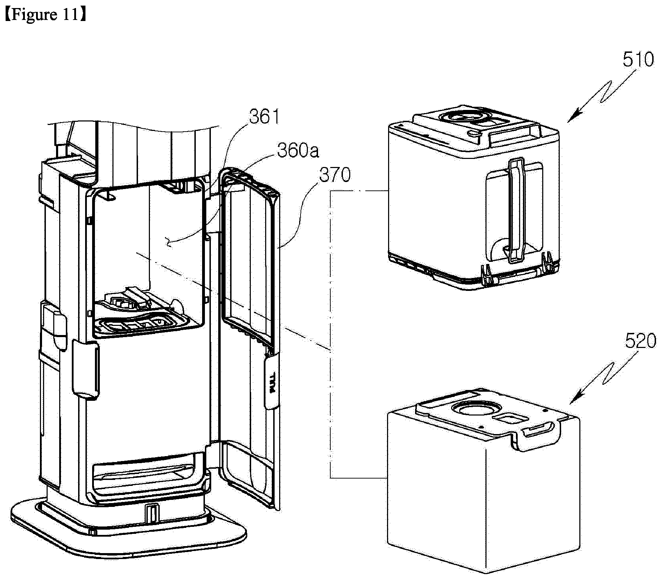

Here, the collector 500 may be separably provided in the chamber 360. The chamber 360 may be configured to be separably provided in the housing 310 or may be configured to be integrally formed with the housing 310. A detailed structure of the chamber 360 will be described below with reference to FIG. 11.

The cleaner station 300 may include the flow path 380.

The flow path 380 is defined as a path through which air and foreign substances which has exited the dust bin 220 of the cleaner 200 flow. The flow path 380 may include the first flow path 381 that connects the dust bin 220 and the collector 500 and may include a second flow path 382 that connects the collector 500 and the dust collection motor 391.

The first flow path 381 may be disposed behind the coupling surface 321. The first flow path 381 may mean a space formed to allow air to flow between the dust bin 220 of the cleaner 200 and the collector 500. For example, the first flow path 381 may be a space formed by being surrounded by structures. For example, the first flow path 381 may be an interior space of a hollow tube.

The first flow path 381 includes a first area 381a and a second area 381b. The first area 381a communicates with an interior space of the dust bin 220 when the cleaner 200 is coupled to the cleaner station 300 and the dust passing hole 321a is opened. The second area 381b communicates the first area 381a and the collector 500 (see FIG. 8).

Accordingly, when the dust collection motor 391 is operated, the dust in the dust bin 220 of the cleaner 200 may flow to the collector 500 through the first flow path 381.

The second flow path 382 may connect the collector 500 and the dust suction module 390. That is, air separated from the dust while passing through the collector 500 may be guided to the dust collection motor 391 through the second flow path 382.

The second flow path 382 may mean a space formed to allow air to flow between the collector 500 and the dust suction module 390. The second flow path 382 may be formed by being surrounded by structures.

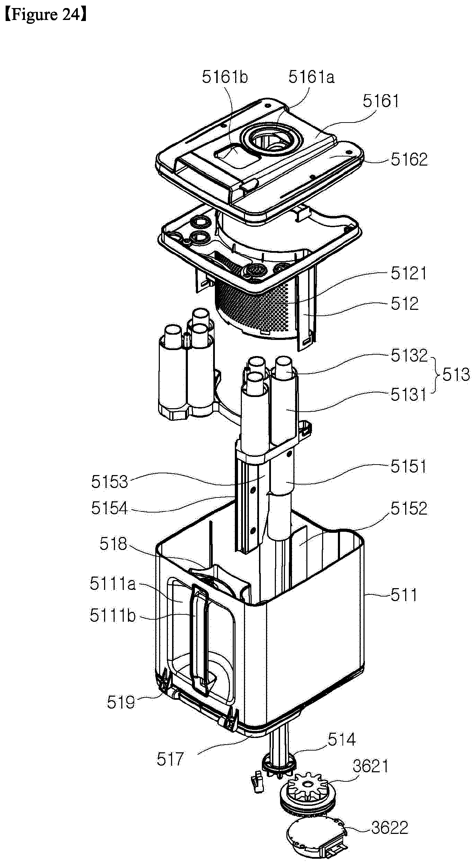

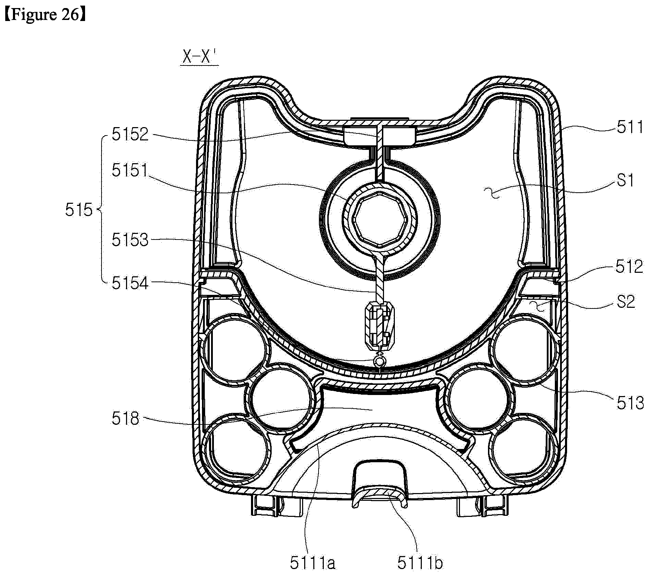

In the embodiment in which the collector 500 is a first collector 510, some areas of the second flow path 382 may be formed within the first collector 510. Said some areas may be referred to as an exhaust flow path 518. The exhaust flow path 518 may be disposed in the front of a collector body 511 (see FIGS. 24 and 26).

The cleaner station 300 may include the dust suction module 390.

Referring back to FIG. 10, the dust suction module 390 may include the dust collection motor 391. The dust collection motor 391 may be disposed below the collector 500. The dust collection motor 391 may generate a suction force in the flow path 380. Through this, the suction force capable of sucking dust into the dust bin 220 of the cleaner 200 is provided.

The dust suction module 390 may further include aHEPAfilter (not shown). The HEPA filter may be disposed at the rear end (based on the air flow path) of the dust collection motor 391. As a result, clean air flows out to the outside of the housing 310.

Meanwhile, although not shown in FIG. 10, the fixing unit 330 and the door unit 340 are disposed adjacent to the coupling portion 320, which has already been described with reference to FIGS. 6 to 8.

The cleaner station 300 may further include the collector 500.

FIG. 11 is a perspective view showing the chamber 360 and the collector 500 formed in a shape that can be coupled to the chamber, according to the embodiment of the present disclosure.

The collector 500 may be separably coupled to the chamber 360. A dust receiving space is provided in the collector 500 in order to collect dust sucked from the inside of the dust bin 220 by the dust collection motor 391.

Meanwhile, the collector 500 according to the embodiment of the present disclosure may be the first collector 510 formed in the form of a bin having the fixed-sized dust receiving space.

Since the first collector 510 is in the form of a "bin", it is easy to clean the inside of the first collector 510. Therefore, there is an advantage that the first collector can be used semi-permanently only the dust received therein is removed. However, there may be users who feel inconvenient to remove the dust and clean the collector, which are tasks that must be separately performed.

Alternatively, the collector 500 according to the embodiment of the present disclosure may be a second collector 520 in the form of a bag having the variably-sized dust receiving space.

The second collector 520 is made of a breathable material that typically allows air to flow out and does not allow dust to flow out. Since the inside of the second collector 520 cannot be cleaned even when the dust contained in the second collector 520 is removed, there is a problem that the second collector 520 must be purchased and replaced periodically. However, the second collector 520 has a simple structure and the second collector 520 can be thrown entirely in a trash can. Therefore, the second collector 520 is advantageous in terms of convenience of users.

The cleaner station 300 according to the embodiment of the present disclosure can be operated even if any of the two types of collectors 510 and 520 is received in the collector receiving space 360a of the chamber 360. That is, the two types of collectors 510 and 520 are compatible.

The user can select the collectors 510 and 520 to be used in consideration of the advantage and disadvantage of each of the above-described collectors 510 and 520 and user's own preferences.

The benefit of compatibility is not limited to the user's preference domain. For example, there is also an advantage that a user who prefers the first collector 510 can temporarily use the second collector 520 as an alternative in the process of cleaning and drying the first collector 510.

Hereinafter, various features of the chamber 360 which allow the different types of collectors 510 and 520 to be compatible will be described.

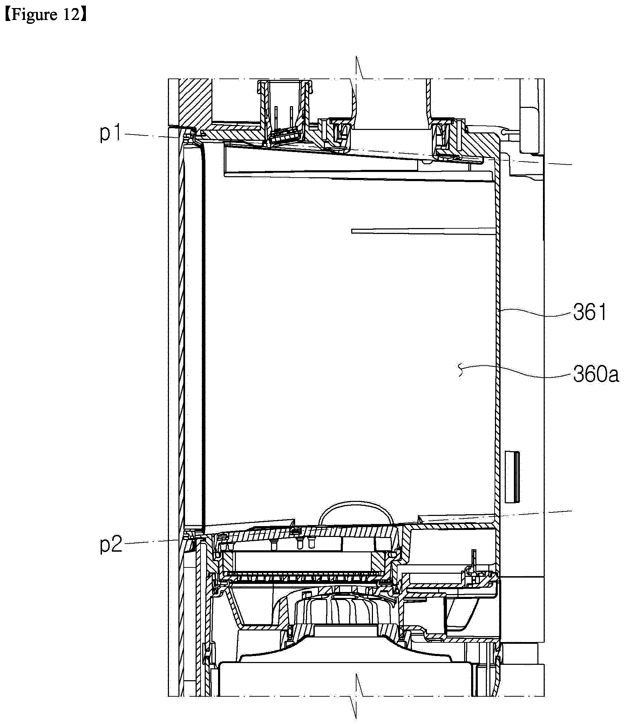

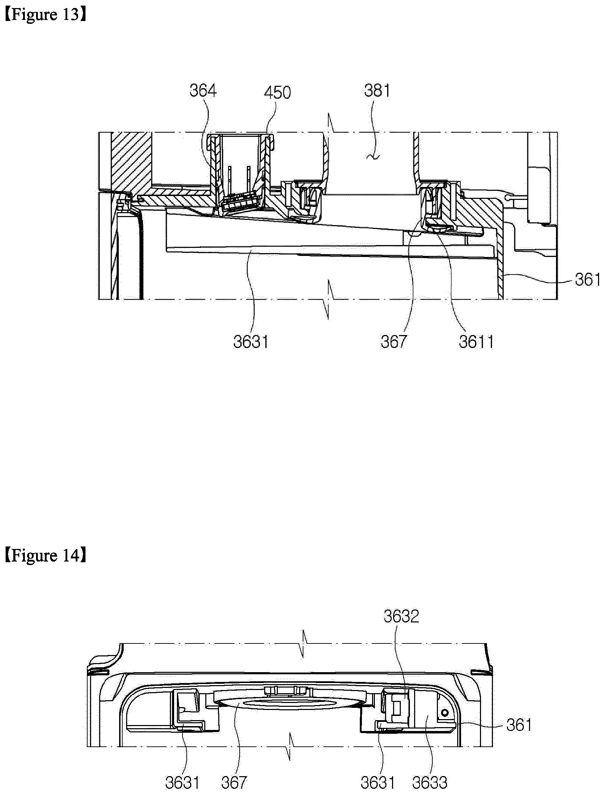

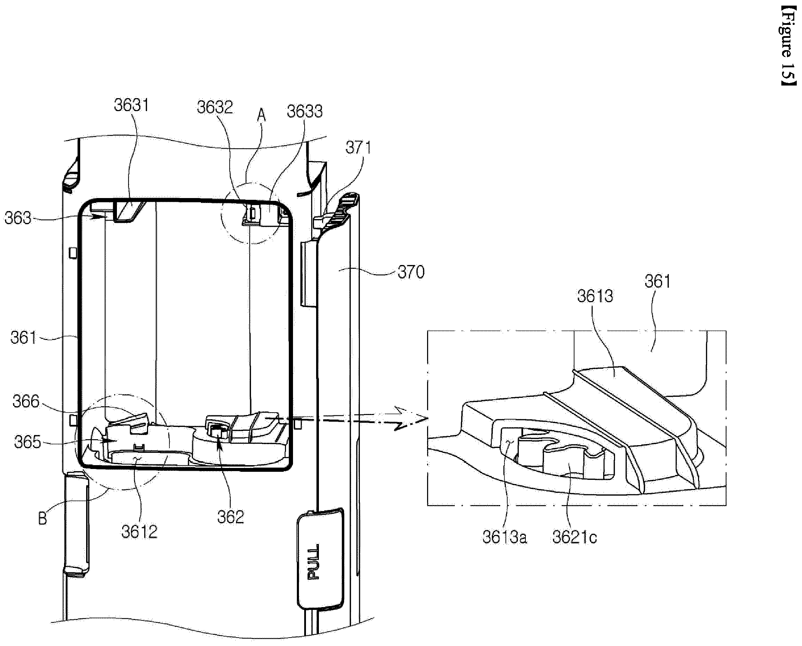

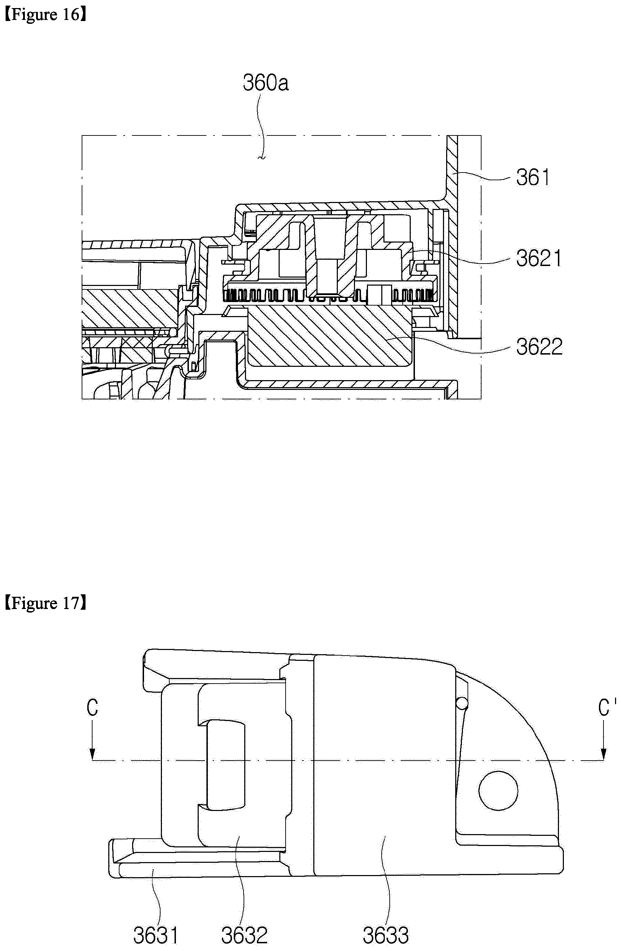

FIG. 12 is a cross-sectional view of the chamber 360 as viewed from the side thereof, in accordance with the embodiment of the present disclosure. FIG. 13 is an enlarged cross-sectional view of an upper side portion of the chamber 360 according to the embodiment of the present disclosure. FIG. 14 is a view of the chamber 360 viewed from the front thereof, in accordance with the embodiment of the present disclosure. FIG. 15 is a perspective view showing an internal structure of the chamber 360 according to the embodiment of the present disclosure. FIG. 16 is s cross-sectional view showing a compression driver 362, in accordance with the embodiment of the present disclosure.

First, referring back to FIG. 11, the chamber 360 may include a chamber body 361.

The chamber body 361 forms the exterior of the collector receiving space 360a. The collector receiving space 360a may have a roughly hexahedral shape.

One side of the chamber body 361 may be opened. The opened one side of the chamber body 361 may be the front. The opened one side of the chamber body 361 may be closed by a housing cover 370.

One side of the housing cover 370 may be rotatably coupled to one side of the housing 300. When the other side of the housing cover 370 rotates in a direction away from the housing 300, one side of the chamber body 361 is opened. Conversely, when the other side of the housing cover 370 rotates in a direction closer to the housing 300, one side of the chamber body 361 may be closed.

The collector receiving space 360a formed by the chamber body 361 may have a cross-sectional area of a front end thereof larger than a cross-sectional area of a rear end thereof.

More specifically, referring to FIG. 12, an imaginary plane p1 connecting the front upper end and the rear upper end of the chamber body 361 may have a rearward-downward inclination.

An imaginary plane p2 connecting the front lower end and the rear lower end of the chamber body 361 may have a rearward-upward inclination.

The upper side of the chamber body 361 is inclined downward toward the rear, and the lower side is inclined upward toward the rear. The collector receiving space 360a has a cross-sectional area of a front end thereof larger than a cross-sectional area of a rear end thereof.

Through such a structure, there is an advantage that the fixed-shaped first collector 510 can be easily inserted into the collector receiving space 360a.

An air inlet 3611 may be provided in the chamber body 361.

The air inlet 3611 passes air introduced through the first flow path 381. The air inlet 3611 may be provided in the top surface of the chamber body 361. Alternatively, the air inlet 3611 may be provided at the end of a hollow tube integrally connected to the chamber body 361.

The air inlet 3611 is disposed to vertically overlap a below-described dust inlet of the collector 500. That is, the air and dust which have passed through the first flow path 381 pass through the air inlet 3611 and the dust inlet in turn and are introduced into the dust receiving space of the collector 500.

An inlet sealing member 367 may be disposed between the air inlet 3611 and the first flow path 381. The inlet sealing member 367 seals between the air inlet 3611 and the first flow path 381 to prevent air and dust from leaking into a space other than the chamber body 361. The inlet sealing member 367 may be disposed to surround the circumference of the air inlet 3611.

As described above, the chamber body 361 has an inclined structure where it is generally inclined downward from the front upper end to the rear upper end. Some areas of the top surface of the chamber body 361 may include a surface inclined rearward and downward at a predetermined angle.

The air inlet 3611 formed in the top of the chamber body 361 may also have its open cross-section inclined downward from the front to the rear.

An air outlet 3612 may be provided in the chamber body 361.

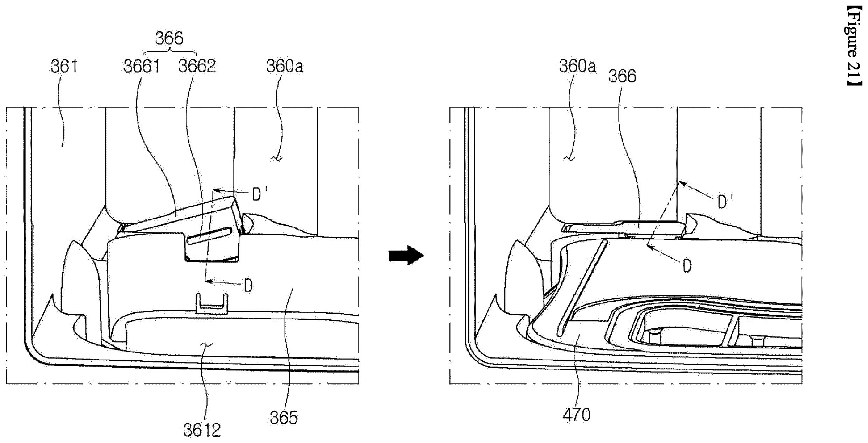

The air outlet 3612 passes air in which dust is filtered while passing through the collector 500. The air outlet 3612 may be provided in a lower portion of the chamber body 361. The air outlet 3612 may be provided below a mounting wall of a below-described filter mounting portion 365 (see FIG. 21).

The air outlet 3612 is connected to the dust suction module 390. That is, the air passing through the air outlet 3612 may be finally exhausted to the outside of the housing through the dust suction module 390.

Referring to FIGS. 11 and 15, the chamber 360 may include the compression driver 362.

The compression driver 362 is configured to generate power to rotate the compression rotating portion 515 that compresses the dust collected in the first collector 510. The compression rotating portion 515 will be described later.

The compression driver 362 may be disposed in the lower portion of the chamber body 361. More specifically, the compression driver 362 may be disposed in an outer lower portion of the chamber body 361.

A detailed configuration of the compression driver 362 will be described with more reference to FIG. 16 as follows.

The compression driver 362 may include a compression motor 3622. The compression motor 3622 may be disposed outside the collector receiving space 360a. That is, the compression motor 3622 may be disposed outside the chamber body 361.

The compression motor 3622 generates power for rotating the compression rotating portion 515. A motor capable of performing a forward rotation and a reverse rotation is provided as the compression motor 3622. In other words, a motor capable of performing a bidirectional rotation is used as the compression motor 3622.

As such, in order to enable the forward and reverse rotations of the compression motor 3622, a synchronous motor can be used as the compression motor 3622. Such a synchronous motor is configured to be capable of the forward and reverse rotations by the motor itself, and if a force applied to the compression motor 3622 is greater than or equal to a set value when the compression motor 3622 rotates in one direction, the rotation direction of the compression motor 3622 is changed to the other direction.

Here, the force applied to the compression motor 3622 is a resistance force (torque) generated by that a below-described rotating plate 5153 compresses the dust. When the resistance force reaches a set value, the rotation direction of the compression motor 3622 is changed.

Technologies for other synchronous motors are commonly known in the field of a motor technology. Therefore, detailed descriptions thereof will be omitted.

The compression driver 362 may further include a drive gear 3621.

The drive gear 3621 is rotated by the power of the compression motor 3622. To this end, the drive gear 3621 may be connected to a motor shaft of the compression motor 3622. The drive gear 3621 may be coupled to the top of the compression motor 3622.

The drive gear 3621 may be disposed outside the collector receiving space 360a. That is, the drive gear 3621 may be disposed outside the chamber body 361.

Here, some areas of the bottom surface of the chamber body 361 may protrude upward to form a drive gear receiving portion 3613 (see FIG. 15).

A gear passing hole 3613a that exposes at least a portion of the drive gear 3621 toward the inside of the collector receiving space 360a is formed in the lower portion of the chamber body 361. More specifically, the gear passing hole 3613a may be formed to pass through a side surface of the drive gear receiving portion 3613. A gear teeth 3621c of the drive gear 3621 may be exposed through the gear passing hole 3613a (see FIG. 15).

Meanwhile, the drive gear 3621 and the compression motor 3622 are disposed outside the collector receiving space 360a. Therefore, considering that the collector receiving space 360a is periodically opened, there is an effect that the drive gear 3621 and the compression motor 3622 are not exposed to contamination.

The drive gear 3621 may transmit rotation power to the compression rotating portion 515.

The compression rotating portion 515 is a concept including one or more components that rotate in order to compress the dust collected in the dust receiving space of the first collector 510.

A detailed structure thereof will be described later. The rotating plate 5153 included in the compression rotating portion 515 rotates and collects the dust. The rotating plate 5153 may compress the dust collected between the fixed plate 5152 and the fixed plate 5152 while rotating so as to be close to the fixed plate 5152 fixedly arranged on one side of the first collector 510.

The first collector 510 may include a transmission gear 514.

The transmission gear 514 may be configured to be coupled to the compression rotating portion 515 and may be disposed between the compression driver 362 and the compression rotating portion 515 to transmit the rotation power.

More specifically, the transmission gear 514 may be disposed outside and below the first collector 510. When the first collector 510 is inserted into the collector receiving space 360a, gear teeth 5142 of the transmission gear 514 may be meshed with the gear teeth 3621c of the drive gear 3621 exposed by the gear passing hole 3613a.

Accordingly, the rotation power generated by the compression motor 3622 may be transmitted to the compression rotating portion 515 via the drive gear 3621 and the transmission gear 514.

Meanwhile, the drive gear 3621 is received in the drive gear receiving portion 3613, and the drive gear receiving portion 3613 is formed to protrude upward from the lowermost surface of the collector receiving space 360a. Therefore, the user can easily cause the transmission gear 514 and the transmission gear 514 to mesh with each other even only by pushing the first collector 510 in horizontally.

Referring to FIGS. 14 and 15, the chamber 360 may include a rail portion 363.

The rail portion 363 may be disposed on the upper portion of the chamber body 361. More specifically, the rail portion 363 may be disposed in the inner upper portion of the chamber body 361.

The rail portion 363 is configured to allow at least a portion of the second collector 520 to be fitted and coupled thereto. The second collector 520 may slidably move along the rail portion 363. The second collector 520 may be supported while being spaced apart from the bottom surface of the chamber body 361 and being suspended from the rail portion 363.

As described above, the second collector 520 is a bag-type collector 500 of which the shape of the dust receiving space is variable.

Here, a dust bag 521 included in the second collector 520 is inflated by suction driving of the dust collection motor 391. Then, the dust may be introduced to the inside of the dust bag 521 from the first flow path 381 by a negative pressure produced by the inflation of the dust bag 521.

In the embodiment of the present disclosure, the dust bag 521 that is a component of the second collector 520 is made of a breathable material. That is, the dust bag 521 is made of a material that typically allows air to flow out and does not allow dust of a certain size or more to flow out. Accordingly, as long as the suction driving of the dust collection motor 391 continues, the dust is continuously sucked into the dust bag 521.

In a state where the dust bag 521 is maximally inflated, when there is no spaced distance between the lowermost end of the dust bag 521 and the inner bottom surface of the chamber body 361, the force for sucking the dust is reduced. Accordingly, in order that the suction force can be evenly transmitted to the dust bag 521, it is preferable that the dust bag 521 be spaced apart from the bottom surface of the chamber body 361 by a predetermined distance even in the state where the dust bag 521 is maximally inflated.

In the embodiment of the present disclosure, the rail portion 363 is provided, which has a structure that leaves a space between the bottom surface of the chamber body 361 and the second collector 520 and supports the second collector 520. Accordingly, the first collector 510 and the second collector can be compatible with each other.

The rail portion 363 may include a rail body 3631.

Referring to FIGS. 13 to 15, the rail body 3631 may form a space in which the second collector 520 slides. The rail body 3631 may be formed to extend from the front to the rear of the chamber body 361. The rail body 3631 may be disposed on the left and right sides, respectively. The rail body 3631 may be formed to have a left-and-right symmetric structure.

The rail body 3631 is opened toward the center of the chamber body 361 and may be provided in a shape similar to a "U"-shape rotated by 90 degrees. From another point of view, the rail body 3631 may be provided in a shape similar to a quadrangular shape having one open side based on the rail body 3631 disposed on the left side.

However, the above-described shape of the rail body 3631 is an example, and the shape of the rail body 3631 can be changed in response to an appropriate structure capable of supporting the second collector 520 over the chamber body 361.

As with the top surface of the chamber body 361, the top surface of the rail body 3631 disposed on the top surface of the chamber body 361 may be formed to be inclined. Unlike this, the bottom surface of the rail body 3631 may be formed parallel to the ground. (See FIG. 13)

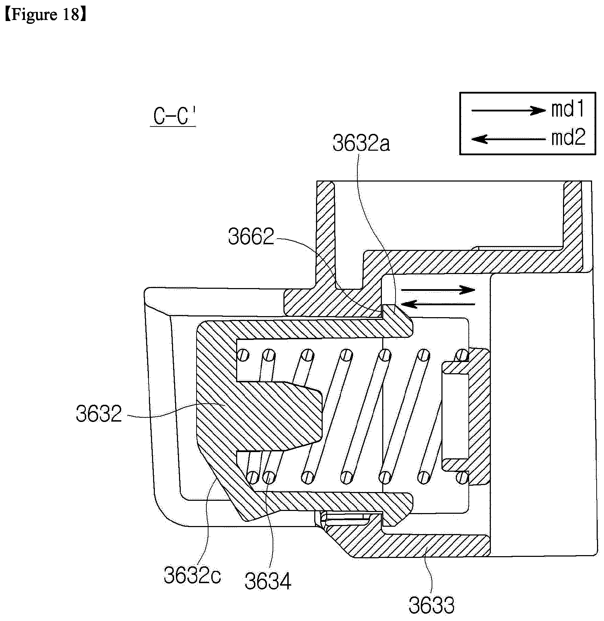

Hereinafter, a detailed structure of the rail portion 363 will be further described with reference to FIGS. 17 and 18.

FIG. 17 is an enlarged view of a part "A" of FIG. 15. FIG. 18 is a cross-sectional view taken along line C-C' of FIG. 17.

Referring to FIGS. 17 and 18 together with FIG. 15, the rail portion 363 may further include an interference protrusion 3632.

The interference protrusion 3632 may be disposed at a first position (Position 1) that interferes with the housing cover 370 in front of the rail body 3631.

More specifically, the first position may mean a position that prevents the housing cover 370 from closing the opened one side of the chamber body 361.

From another point of view, the first position may mean a position that shields an insertion progress path of some components included in the first collector 510 and the second collector 520 when the first collector 510 or the second collector 520 is inserted into the collector receiving space 360a.

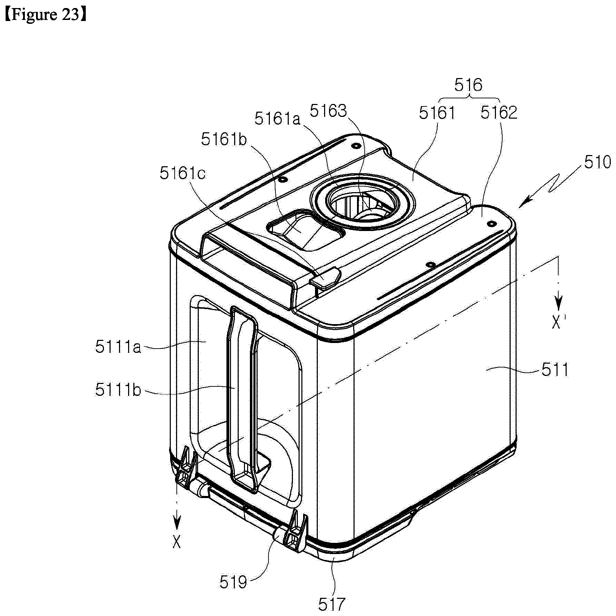

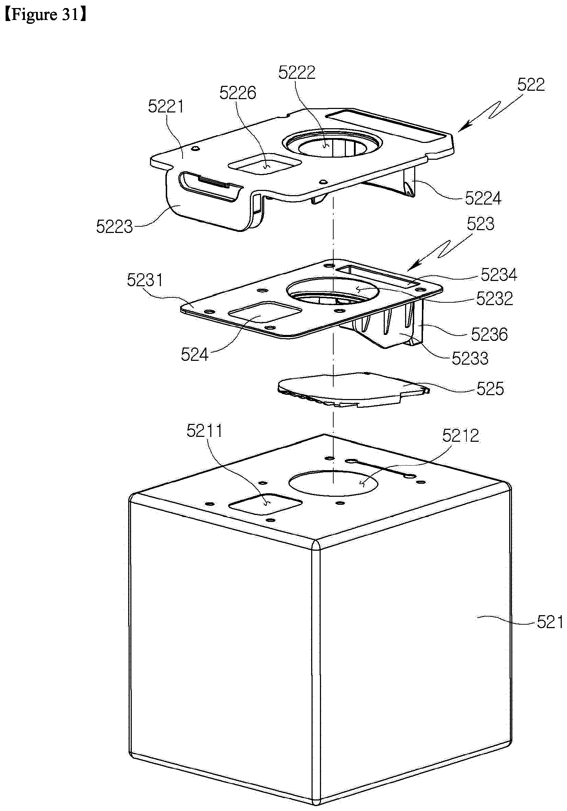

Here, the above some components of which the insertion progress paths are shielded may be an outer plate 522 to be described later, in the case of the first collector 510 (see FIG. 31). In the case of the second collector 520, the above some components may be a rib 5161c formed to protrude from a body cover 516 (refer to FIG. 23).

The interference protrusion 3632 may be moved to a second position (Position 2) that avoids the interference with the housing cover 370 by contact with the first collector 510 (specifically, the outer plate 522) or the second collector 520 (specifically, the rib 5161c).

Meanwhile, a cover protrusion 371 may protrude from the housing cover 370.

When the housing cover 370 rotates in order to close the opened one side of the chamber body 361, the cover protrusion 371 may be disposed at a position where the housing cover 370 comes in contact with the aforementioned interference protrusion 3632 (FIG. 15). That is, what the interference protrusion 3632 interferes with the housing cover 370 means that the interference protrusion 3632 and the cover protrusion 371 are in contact with each other.

The rail portion 363 may further include a protrusion guide 3633.

Referring to FIGS. 17 and 18 continuously, the protrusion guide 3633 may be coupled to the interference protrusion 3632. The protrusion guide 3633 may guide the movement of the interference protrusion 3632.

More specifically, the protrusion guide 3633 has a space formed therein to receive the interference protrusion 3632. A catching protrusion 3662 may be formed within the space and may be caught by and coupled to a hook 3632a formed at the end of the interfering protrusion 3632.

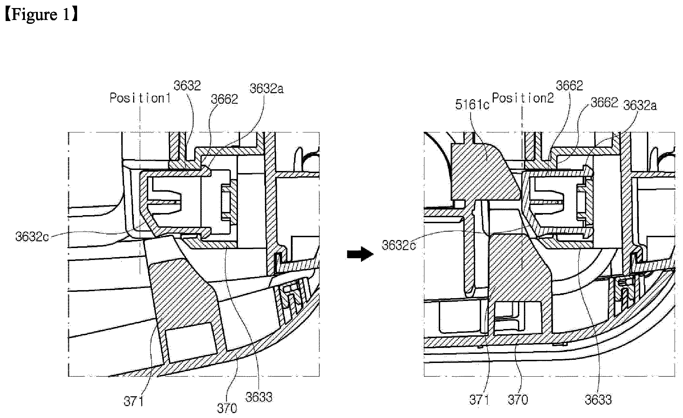

The interference protrusion 3632 may be disposed at the first position in a state where the hook 3632a and the catching protrusion 3662 are in contact with each other. As the interference protrusion 3632 moves from the first position to the second position, the contact between the hook 3632a and the catching protrusion 3662 may be released. (See FIG. 19)

The rail portion 363 may further include a restoring member 3634.

Referring to FIG. 18, the restoring member 3634 may be coupled to a space formed within the interference protrusion 3632. A direction in which the interference protrusion 3632 moves from the first position to the second position is defined as a first moving direction "md1", and a direction opposite to the first moving direction is defined as a second moving direction "md2". The restoring member 3634 may provide the interference protrusion 3632 with an elastic force toward the second moving direction, and the interference protrusion 3632 may be moved in the second moving direction until the hook 3632a and the catching protrusion 3662 are contacted by the restoring member 3634.

Referring to FIG. 19, the operation of the interference protrusion 3632 when the collector 500 is inserted into the collector receiving space 360a and when not inserted will be described.

FIG. 19 is a view describing the movement of the interference protrusion 3632 and thus a position relationship with the interference protrusion and the housing cover 370, in accordance with the embodiment of the present disclosure.

The figure on the left of FIG. 19 shows that the interference protrusion 3632 is in the first position and the cover protrusion 371 and the interference protrusion 3632 are in contact with each other. Here, as the cover protrusion 371 comes into contact with the interference protrusion 3632, the direction in which the force is applied to the interference protrusion 3632 and the direction in which the interference protrusion 3632 moves from the first position to the second position may form a predetermined angle.

There is no component of the direction of the force that moves the interference protrusion 3632 from the first position to the second position. Therefore, the movement of the interference projection 3632 cannot be made by the pressing force of the cover projection 371, and the interference projection 3632 prevents the housing cover 370 from rotating any longer. Accordingly, the chamber body 361 is cannot be closed.

The figure on the right of FIG. 19 shows that the interference protrusion 3632 is in the second position and the cover protrusion 371 does not come into contact with the interference protrusion 3632. The housing cover 370 may moves rotationally until the housing cover 370 closes fully the opened one side of the chamber body 361.

Here, it is the first collector 510 or the second collector 520 that moves the interference protrusion 3632 from the first position to the second position. When the first collector 510 or the second collector 520 is inserted into the collector receiving space 360a, some components of the first collector 510 or the second collector 520 may push the interference protrusion 3632 to the second position.

The interference protrusion 3632 may be provided with an inclined portion 3632c that comes into contact with a portion of the collector 500.

Referring to FIG. 15, the chamber 360 may further include a filter mounting portion 365.

The filter mounting portion 365 may be disposed in the lower portion of the chamber body 361. The filter mounting portion 365 may mean a filter mounting space and a mounting wall. A pre-filter module 470 that filters the air which has passed through the collector 500 is coupled in an attachable and detachable manner to the filter mounting space. The mounting wall surrounds the filter mounting space.

Here, the above-described air outlet 3612 is formed at the lower end of the mounting wall. In another view, the air outlet 3612 is formed at the lower end of the filter mounting space.

If the pre-filter module 470 is coupled to the filter mounting portion 365, the air exiting the collector 500 and flowing to the air outlet 3612 passes through the pre-filter module 470 and then moves to the dust suction module 390 via the air outlet 3612.

If the pre-filter module 470 is not coupled to the filter mounting portion 365, the air which has exited the collector 500 passes through the air outlet 3612 as it is and moves to the dust suction module 390.

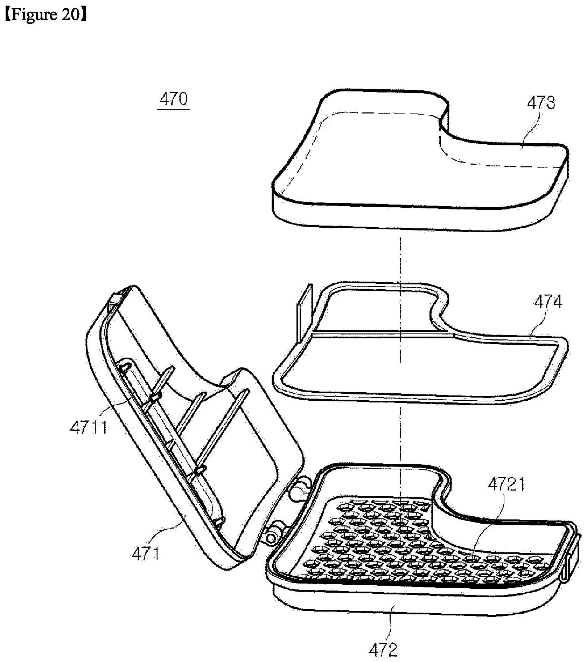

FIG. 20 is a view showing that the pre-filter module 470 is divided into constituents and developed in accordance with the embodiment of the present disclosure.

Referring to FIG. 20, the pre-filter module 470 may include filter cases 471 and 472 and filters 473 and 474.

The filter cases 471 and 472 may include the upper case 471 and the lower case 472, and the upper case 471 and the lower case 472 may be coupled by a hinge. When they rotate about the hinge, a space within the case can be opened and closed.

An air inlet 4711 is formed on the case upper 471.

The air inlet 4711 may be formed on the top surface of the upper case 471 in such a way as to have a shape corresponding to the shape of the exhaust flow path 518 to be described later of the first collector 510. The air which has exited the exhaust flow path 518 of the first collector 510 may be introduced into the filter cases 471 and 472 through the air inlet 4711. The air which has exited the dust bag 521 of the second collector 520 may be introduced into the filter cases 471 and 472 through the air inlet 4711.

An air outlet 4721 is formed on the lower case 472.

The air outlets 4721 may be formed of a plurality of holes passing through the bottom surface of the lower case 472. The air outlets 4721 may be distributed over the entire area of the bottom surface of the case lower 472 such that air can pass uniformly through the filter.

At least one of the filters 473 and 474 may be received in an interior space formed by the upper case 471 and the lower case 472. The filters 473 and 474 may include the first filter 473 made of a sponge material. The filter 473 and 474 may include the second filter 474 made of a non-woven fabric.

A pressing protrusion 4712 may be formed to protrude from one side of the filter cases 471 and 472.

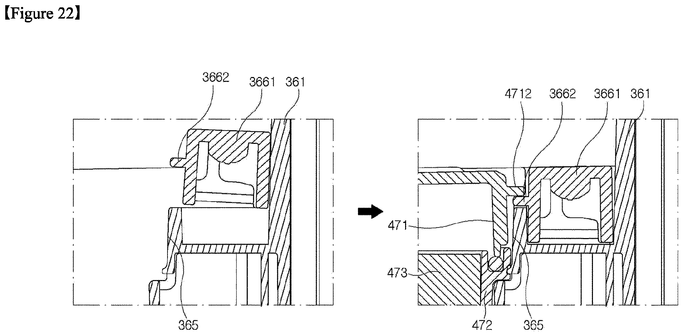

When the pre-filter module 470 is mounted on the filter mounting portion 365, the pressing protrusion 4712 is configured to press a filter detection protrusion 366 to be described later. The pressing protrusion 4712 may be formed in the form of a rib protruding from one surface of the upper case 471 or the lower case 472 (See FIG. 22).

Referring to FIG. 15, the chamber 360 may further include the filter detection protrusion 366.

The filter detection protrusion 366 may be disposed in the lower portion of the chamber body 361. More specifically, the filter detection protrusion 366 may be disposed adjacent to the mounting wall of the filter mounting portion 365.

One side of the filter detection protrusion 366 may remain protruding toward the collector receiving space 360a in a state where the pre-filter module 470 is not coupled to the filter mounting portion 365.

To this end, although not shown, a pressing means for applying a force in a direction in which the one side protrudes may be coupled to the filter detection protrusion 366. For example, the pressing means may be an elastic body such as a spring.

As such, when one side of the filter detection protrusion 366 protrudes toward the collector receiving space 360a, the one rear side of the first collector 510 and the filter detection protrusion 366 cause interference with each other, so that it is impossible to completely insert the first collector 510 into the collector receiving space 360a.

On the contrary to this, since the second collector 520 is maintained spaced apart from the lower end of the chamber body 361 even when the dust bag 521 is fully inflated, the second collector 520 is inserted without being interrupted by the filter detection protrusion 366.

Through this configuration, the user cannot use the first collector 510 without mounting the pre-filter module 470 on the filter mounting portion 365. When the user separates the pre-filter module 470 and inserts the first collector 510 into the chamber body 361 while forgetting separating the pre-filter module 470, the first collector 510 is caught by the filter detection protrusion 366 and would not be completely inserted. After mounting the pre-filter module 470 on the filter mounting portion 365, the user can insert the first collector 510 into the chamber body 361 again.

Meanwhile, in a state where the pre-filter module 470 is coupled to the filter mounting portion 365, one side of the filter detection protrusion 366 may be pressed by the pre-filter module 470. In this case, the filter detection protrusion 366 may be moved in a direction to avoid interference with the first collector 510.

FIG. 21 shows an enlarged part "B" of FIG. 15 and is a view for describing a movement of the filter detection protrusion 366 depending on whether the pre-filter module 470 is mounted or not. FIG. 22 is a cross-sectional view taken along line D-D' of FIG. 21.

Referring to FIGS. 21 and 22, the filter detection protrusion 366 may include a protrusion body 3661 and the catching protrusion 3662 which protrudes from the outer surface of the protrusion body 3661. Here, a space in which the above-described pressing means (not shown) is disposed may be formed within the protrusion body 3661.

Referring to the figure on the left of FIG. 21 and the figure on the left of FIG. 22, the figures show a state where the pre-filter module 470 is not mounted on the filter mounting portion 365. One side of the filter detection protrusion 366 remains protruding toward the collector receiving space 360a.

The figure on the right of FIG. 21 shows a state where the pre-filter module 470 is mounted on the filter mounting portion 365. Referring to the figure on the right of FIG. 22, the catching protrusion 3662 of the filter detection protrusion 366 is pressed by the pressing protrusion 4712.

Here, as in the embodiment of FIG. 22, the pressing protrusion 4712 may be formed to protrude from one surface of the upper case 471. As the pre-filter module 470 is mounted on the filter mounting portion 365, the pressing protrusion 4712 may come into contact with the stopping protrusion 3662, may invade the collector receiving space 360a, and then may press downward one side of the filter detection protrusion 366 which remains protruding.

Accordingly, the first collector 510 can be completely inserted into the chamber body 361 without interference.

On the other hand, the necessity of the above-described filter detection protrusion 366 is related to the fact that the dust separation means of the first collector 510 and the dust separation means of the second collector 520 are different from each other.

A mesh net 5121 and a cyclone 513 may be included as the dust separation means of the first collector 510.

A dust bag 521 made of a breathable material may be included as the dust separation means of the second collector 520.

In the case of the second collector 520, structurally, dust of a certain size or more cannot pass through the dust bag 521. Since the dust bag 521 is able to perform a function of a pre-filter by itself, the pre-filter module 470 is mounted according to the selection of the user and is not indispensable.

However, in the case of the first collector 510, although it is possible to generally separate fine dust through the dust collection operation of the cyclone 513, it is impossible to structurally completely filter out the fine dust, just like the dust bag 521 of the first collector 510.

Therefore, in order to prevent fine dust from entering the dust collection motor 391, the pre-filter module 470 must be disposed in the path through which the air which has passed through the first collector 510 flows into the dust collection motor 391.

According to the embodiment of the present disclosure, there is an effect that the use of the first collector 510 alerts the user to pay attention to the unequipped pre-filter module 470.

Also, according to the embodiment of the present disclosure, the second collector 520 can be used regardless of whether the pre-filter module 470 is mounted or not, thereby reducing the filter purchase cost according to the user's selection.

Referring to FIG. 13, the chamber 360 may further include a sterilization module mounting portion 364.

The sterilization module mounting portion 364 may be disposed on the chamber body 361. The sterilization module mounting portion 364 may be disposed to be spaced apart from the air inlet 3611. The sterilization module mounting portion 364 may include a sterilization module mounting space and a mounting wall. The sterilization module mounting space is formed by bending upward a portion of the top surface of the chamber body 361. The mounting wall surrounds the mounting space.

The sterilization module mounting portion 364 may be equipped with a sterilization module 450 that is provided to irradiate ultraviolet light toward the collector receiving space 360a.

The sterilization module 450 is configured to sterilize the dust collected by the collector 500. The sterilization module 450 may include a light source and a protection panel. The light source emits sterilization light. The protection panel is disposed below the light source and protects the light source.

In a possible embodiment, the light source and the protection panel may be mounted on the sterilization module mounting portion 364 in a form that is received in a separately provided housing. Alternatively, in a possible embodiment, the light source and the protection panel may be mounted in the sterilization module mounting space in a form that is directly received in the sterilization module mounting space.

As such, as long as the sterilization module 450 and the sterilization module mounting portion 364 are arranged and coupled such that the light source of the sterilization module 450 emits the sterilization light toward the collector receiving space 360a, the arrangement form is not limited to any one embodiment.

Here, the light source may include at least one light emitting diode (LED) capable of emitting sterilization light having sterilizing power capable of removing bacteria. The sterilization light emitted by the light source may have a wavelength that varies depending on the type of light emitting diode.

For example, the light source may be a light emitting diode that emits ultraviolet light with a UV-C wavelength range. The UV light is divided into UV-A (315 nm to 400 nm), UV-B (280 nm to 315 nm), and UV-C (200 nm to 280 nm) depending on the wavelength. Among them, the UV light in the UV-C region damages DNA double helix and inhibit the growth of microorganisms.

Alternatively, for another example, the light source may be a light emitting diode that emits visible light with a wavelength of 405 nm. Blue light with a wavelength of 405 nm has a wavelength in the boundary region between visible light and ultraviolet light and has proved its sterilizing power.

The protection panel may be disposed to be spaced apart from the light source by a predetermined distance in order to prevent the light source from being damaged. Here, the protection panel may be made of a material that maximizes the transmittance of the light source. For example, the protection panel may be made of quartz. It is known that the quartz does not interfere with the transmission of ultraviolet light in the UV-C region.

In the embodiment of the present disclosure, the sterilization module 450 and the sterilization module mounting portion 364 are provided. Therefore, even when the dust sucked from the dust bin 220 of the cleaner 200 is stored in the collector 500 for a long period of time, there is an advantage that the cleaner station 300 can be hygienically managed.

In addition, in the embodiment of the present disclosure, since the sterilization module mounting portion 364 is disposed in the chamber 360, there is an advantage that the user can use the sterilization function no matter what type of collector 500 is selected.

Hereinafter, a detailed structure of the first collector 510 will be described.

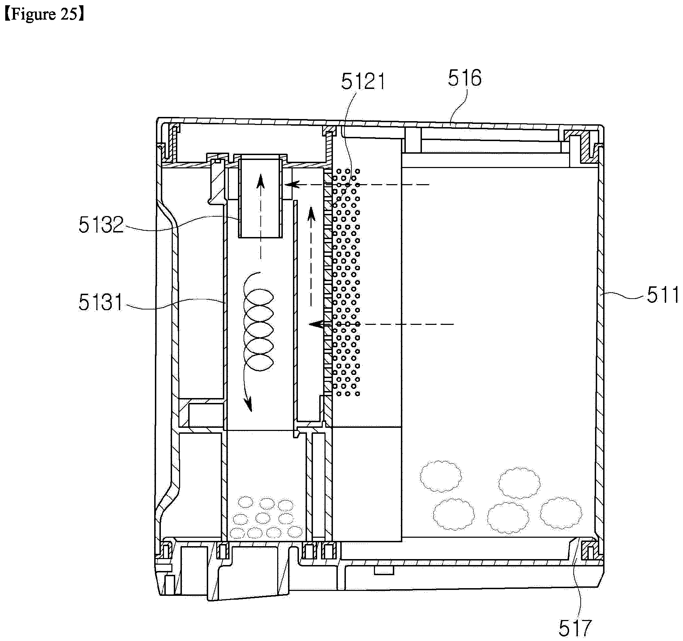

FIG. 23 is a perspective view of the first collector 510 according to the embodiment of the present disclosure. FIG. 24 is a view showing that components related to the first collector 510 are divided and developed in accordance with the embodiment of the present disclosure. FIG. 25 is a view showing a dust separation process of the cyclone 513 in accordance with the embodiment of the present disclosure. FIG. 26 is a cross-sectional view taken along line X-X' of FIG. 23.

Referring to FIGS. 23 to 26, the first collector 510 may include the collector body 511, a collector inner wall 512, and the cyclone 513.

The collector body 511 forms the exterior of the dust receiving space. In a possible embodiment, the collector body 511 may generally have a hexahedral shape. In another possible embodiment, the collector body 511 may generally have a cylindrical shape.

The first collector 510 may further include the body cover 516 that is provided to cover the open top of the first collector 510 body.

The body cover 516 may have a shape corresponding to the shape of the rail portion 363.

More specifically, the body cover 516 may include a first cover portion 5161 and a second cover portion 5162.

The top surface of the first cover portion 5161 may be formed to be inclined rearward and downward. Accordingly, the first collector 510 may have a smaller height to the rear upper portion thereof and may have a greater height to the front upper portion thereof.

Meanwhile, as described above, in the collector receiving space 360a formed by the chamber body 361, the cross-sectional area of the front end may be larger than the cross-sectional area of the rear end.

That is, the rear of the first collector 510, which starts to be inserted into the collector receiving space 360a, has a lower height, and the front end of the collector receiving space 360a, which is an entrance into which the first collector 510 is inserted, has a greater height. Accordingly, the first collector 510 can be easily inserted into the collector receiving space 360a.

The second cover portion 5162 may be connected to both left and right sides of the first cover portion 5161. The second cover portion 5162 may be formed to extend from the side bottom end of the first cover portion 5161 in the horizontal direction. That is, the top surface of the first cover portion 5161 and the top surface of the second cover portion 5162 may form a step difference such that the top surface of the first cover portion 5161 is at a higher position.

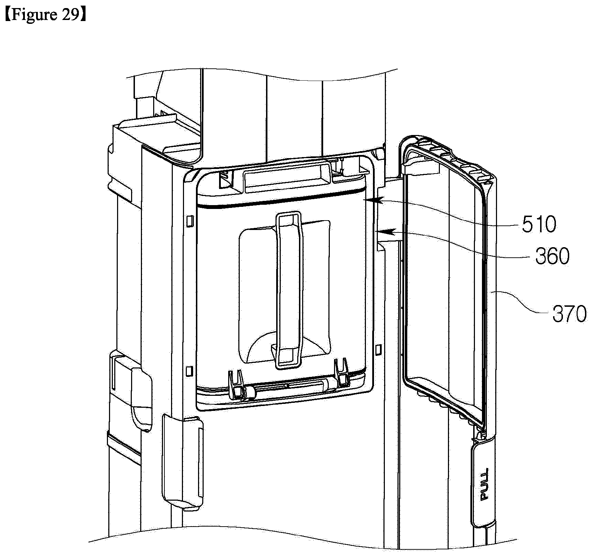

In a state where the first collector 510 is inserted into the chamber body 361, a side surface of the first cover portion 5161 is disposed to face a side surface of the rail body 3631. More specifically, left and right side surfaces of the first cover portion 5161 are disposed to face the side surfaces of the left and right rail bodies 3631. From another point of view, the first cover portion 5161 is disposed between the left and right rail bodies 3631 (see FIG. 29).

In a state where the first collector 510 is inserted into the chamber body 361, the top surface of the second cover portion 5162 is disposed to face the bottom surface of the rail body 3631.

A transmissive panel 5161b may be disposed on the top surface of the first cover portion 5161.

When the first collector 510 is inserted into the chamber body 361, the transmissive panel 5161b is disposed at a position corresponding to the position at which the sterilization module 450 is disposed. That is, when the first collector 510 is inserted into the chamber body 361, the sterilization module 450 and the transmissive panel 5161b are disposed to face each other.

The transmissive panel 5161b is made of a material through which the sterilization light emitted from the sterilization module 450 can transmit toward the inside of the collector body 511. For example, the transmissive panel 5161b may be made of a poly methyl methacrylate (PMMA) material.

A dust inlet 5161a through which dust as well as air is introduced from the first flow path 381 may be formed on the top surface of the first cover portion 5161. For example, the dust inlet 5161a may have a circular shape. The first flow path 381 is connected to the top side of the dust inlet 5161a. Accordingly, air sucked by the suction force of the dust collection motor 391 may be introduced into the collector body 511. The dust inlet 5161a may be disposed in the upper portion of a first dust receiving space S1 to be described later.

Based on the state where the first collector 510 is inserted into the chamber body 361, the dust inlet 5161a and the air inlet 3611 of the chamber body 361 may be positioned to face each other.

Here, since the dust inlet 5161a is formed in the top surface formed to be inclined rearward and downward of the first cover portion 5161, the cross section of the dust inlet 5161a is also inclined rearward and downward. The foregoing has described that the cross-section of the air inlet 3611 is also inclined rearward and downward. Since the air inlet 3611 and the dust inlet 5161a have an inclination in the same direction, they can be sealed without being spaced apart from each other.

An inlet cover 5163 for opening and closing the dust inlet 5161a may be coupled to an inner top surface of the first cover portion 5161. The inlet cover 5163 is configured to close or open the dust inlet 5161a. The inlet cover 5163 may be provided in a shape corresponding to the shape of the dust inlet 5161a and may be coupled to one side of the dust inlet 5161a.

The inlet cover 5163 may be opened by the suction force of the dust collection motor 391. That is, the inlet cover 5163 may be opened toward an interior space (specifically, the first dust receiving space S1) of the collector body 511.

The inlet cover 5163 may maintain the dust inlet 5161a to be closed when the dust collection motor 391 is not driven. When the dust collection motor 391 starts to run, the inlet cover 5163 may open the dust inlet 5161a.

To this end, the inlet cover 5163 may be provided with a means for applying a restoring force in a direction to close the dust inlet 5161a. The restoring force applying means may be, for example, an elastic member such as a spring.

Through this configuration, the inlet cover 5163 maintains the dust inlet 5161a to be closed all the time in a state where no suction force is applied. Also, odors, contamination, bacteria, etc., that may occur within the first collector 510 can be prevented from spreading to the first flow path 381.

The rib 5161c may be formed to protrude from an outer surface of the first cover portion 5161.

Here, the outer surface may refer to a surface that connects the top surface of the first cover portion 5161 and the top surface of the second cover portion 5162.

The rib 5161c may be formed in the front of the outer surface of the first cover portion 5161. The rib may protrude in a direction parallel to the top surface of the second cover portion 5162. As the first collector 510 is inserted into the chamber body 361, the rib 5161c may be inserted into a sliding space of the rail portion 363 (specifically, the rail body 3631).

As the first collector 510 is inserted into the chamber body 361, the rib 5161c may push the interference protrusion 3632 disposed at the first position to the second position (see FIG. 19).

The first collector 510 may further include a bottom cover 517 provided to cover the open bottom side of the first collector 510 body.

A collector hinge 519 may be disposed on one side of the edge of the bottom cover 517. When the bottom cover 517 rotates about the collector hinge 519, the inside of the collector body 511 may be opened. Accordingly, the user can remove the dust collected in the collector body 511 by exhausting the dust to the outside.

A handle 5111b may be disposed on the front (or front side) of the collector body 511. The handle 5111b is configured to be gripped by the user such that the first collector 510 can be pulled out to the outside of the chamber body 361. The user can easily pull out the first collector 510 from the chamber body 361 by gripping the handle 5111b and pulling the first collector 510 forward.

A finger recess 5111a may be provided in the collector body 511 such that the user can easily grip the handle 5111b. The finger recess 5111a is formed enough for user's fingers to be fitted therein. The finger recess 5111a is formed in a shape in which the collector body 511 is recessed toward the interior space of the first collector 510.

Through this configuration, the handle 5111b is not formed to excessively protrude to the outside of the collector body 511. That is, this configuration can contribute to the miniaturization of the cleaner station 300.

The collector inner wall 512 may be disposed within the dust receiving space of the collector body 511. The collector inner wall 512 may divide the dust receiving space of the collector body 511 into two separate spaces. The collector inner wall 512 may be disposed in a direction perpendicular to the ground.

The mesh net 5121 may be provided on the collector inner wall 512. For example, the mesh net 5121 may constitute a portion of the collector inner wall 512. That is, when air flows from one side to the other side of the divided dust receiving space, the air may pass through the mesh net 5121.

Meanwhile, a receiving space within the first collector 510 is divided into the first dust receiving space S1 and a second dust receiving space S2 by the collector inner wall 512.

The compression rotating portion 515 may be disposed in the first dust receiving space S1. The cyclone 513 may be disposed in the second dust receiving space S2. Air sucked through the first flow path 381 from the outside first flows into the first dust receiving space S1 and flows into the second dust receiving space S2 through the mesh net 5121.

Through this, relatively large dust may be filtered by the mesh net 5121. The filtered large dust is collected and stored in the lower portion of the first dust receiving space S1.

A plurality of cyclones 513 may be provided. For example, at least two cyclone bodies having a conical shape or a cylindrical shape may be provided.

Referring to FIG. 25, each cyclone body includes an inlet body 5131 into which air which has passed through the mesh net 5121 is introduced. Each cyclone body further includes an outlet body 5132 connected to the exhaust flow path 518.

The exhaust flow path 518 is connected to the dust collection motor 391 side, and constitutes, as described above, a portion of the second flow path 382.

Air is sucked from the outlet body 5132 by the suction force applied to the exhaust flow path 518, and a cyclone flow is generated in the inlet body 5131. By this cyclone flow, fine-sized dust can be filtered from the air that has passed through the mesh net 5121.

The first collector 510 may include the compression rotating portion 515.

The compression rotating portion 515 is configured to compress the dust collected in the first collector 510.

Referring to FIGS. 24 and 26, the compression rotating portion 515 is disposed within the dust receiving space of the collector body 511. More specifically, the compression rotating portion 515 is disposed within the first receiving space S1 of the collector body 511.

The compression rotating portion 515 is movably disposed within the collector body 511. The compression rotating portion 515 may move in a direction to compress the dust collected within the first dust receiving space S1. In the embodiment of the present disclosure, the compression rotating portion 515 may be rotatably disposed in the first dust receiving space S1.

The compression rotating portion 515 may rotate about an axis disposed in the longitudinal direction within the collector body 511 (the first dust receiving space S1). More specifically, the compression rotating portion 515 may include a rotation shaft member 5151, the fixed plate 5152, and the rotating plate 5153.

The rotation shaft member 5151 may be disposed within the collector body 511, that is, in the first dust receiving space S1 in an up and down direction. The rotation shaft member 5151 may be rotated by receiving the power from the compression motor 3622 described above. A central axis of the rotation shaft member 5151 may be coaxial with a central axis of the first dust receiving space S1.

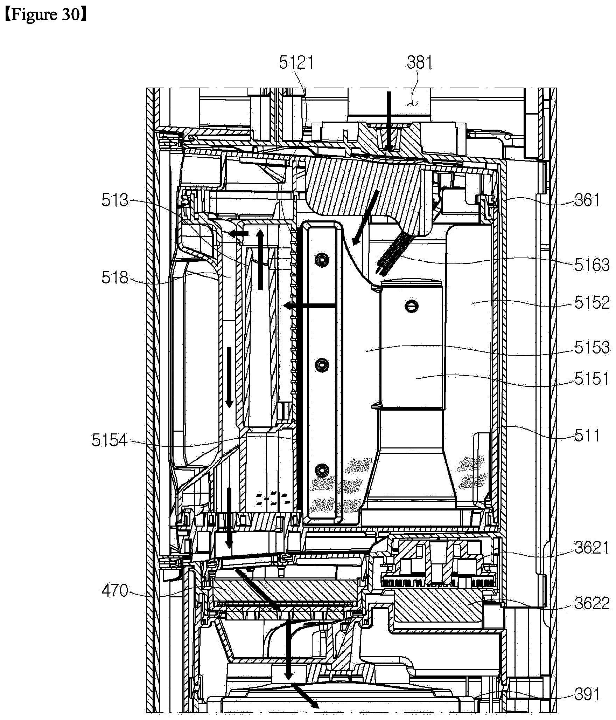

The lower end of the rotation shaft member 5151 can be supported by being connected to the bottom surface of the first dust receiving space S1. The upper end of the rotation shaft member 5151 may be spaced apart from the dust inlet 5161a by a predetermined distance so as not to interfere with the opening of the inlet cover 5163 (see FIG. 30).

The fixed plate 5152 may be fixedly disposed on one side within the first collector 510. More specifically, the fixed plate 5152 may be disposed in the first dust receiving space S1 in an up and down direction and may be fixedly coupled to one side of the inner peripheral surface of the collector body 511 which forms the first dust receiving space S1. The fixed plate 5152 may be in the form of a quadrangular flat plate. The fixed plate 5152 may be disposed opposite to the mesh net 5121.

The fixed plate 5152 may completely or partially shield the first dust receiving space S1, thereby, together with the rotating plate 5153, compressing dust pushed and moved by the rotation of the rotating plate 5153.

The rotating plate 5153 may be disposed to be connected to the outer peripheral surface of the rotation shaft member 5151 and may rotate together with the rotation shaft member 5151. More specifically, the rotating plate 5153 may be disposed between the inner peripheral surface of the collector body 511 constituting the first dust receiving space S1 and the outer peripheral surface of the rotation shaft member 5151 and may rotate.

The shape of the rotating plate 5153 is basically a quadrangular flat plate shape, and may be transformed in the form of avoiding interference with other components disposed in the first dust receiving space S1. For example, in order that the rotating plate 5153 does not interfere with the rotation radius of the inlet cover 5163 when the inlet cover 5163 opens the dust inlet 5161a, a cutout portion may be formed at the upper end of the rotating plate 5153 (See FIG. 30.)

The rotating plate 5153 may rotate in forward and reverse directions. Based on when the first dust receiving space S1 is viewed from the top (that is, when the first collector 510 is viewed from above), a clockwise rotation is defined as a forward rotation, and a counterclockwise rotation is defined as reverse rotation.

When the rotating plate 5153 rotates in the forward direction, one surface of the rotating plate 5153 and one surface of the fixed plate 5152 meet each other to compress the dust. Similarly, when the rotating plate 5153 rotates in the reverse direction, the other surface of the rotating plate 5153 and the other surface of the fixed plate 5152 meet each other to compress the dust. That is, some of the compressed dust is present near the one surface of the fixed plate 5152, and the rest is present in the vicinity of the other surface of the fixed plate 5152.

The compression rotating portion 515 may further include a cleaning member 5154.

Referring to FIGS. 24 and 26, the cleaning member 5154 is coupled to the end of the rotating plate 5153 on the opposite side to where the rotation shaft member 5151 is disposed. That is, one side end of the rotating plate 5153 is coupled to the rotation shaft member 5151 and the other side end is coupled to the cleaning member 5154.

The cleaning member 5154 may be provided to rotate together with the rotating plate 5153 in a state of contacting the mesh net 5121. More specifically, one side edge of the cleaning member 5154 may be disposed to contact with one surface of the mesh net 5121.

Through this, when the cleaning member 5154 rotates together with the rotating plate 5153, the cleaning member 5154 may rotate while scratching the mesh net 5121, and foreign substances adhering to the mesh net 5121 can be removed. The cleaning member 5154 may be, for example, a scrubber made of rubber.

Meanwhile, as described above, the first collector 510 may include the transmission gear 514.

The transmission gear 514 may be coupled to the bottom cover 517 of the first collector 510. When the first collector 510 is inserted into the collector receiving space 360a, the transmission gear 514 and the drive gear 3621 may mesh with each other, and the rotation power of the compression motor 3622 may be transmitted to the compression rotating portion 515 via the drive gear 3621 and the transmission gear 514.

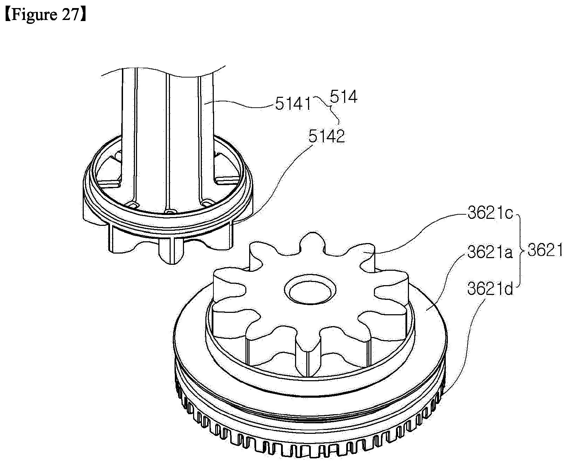

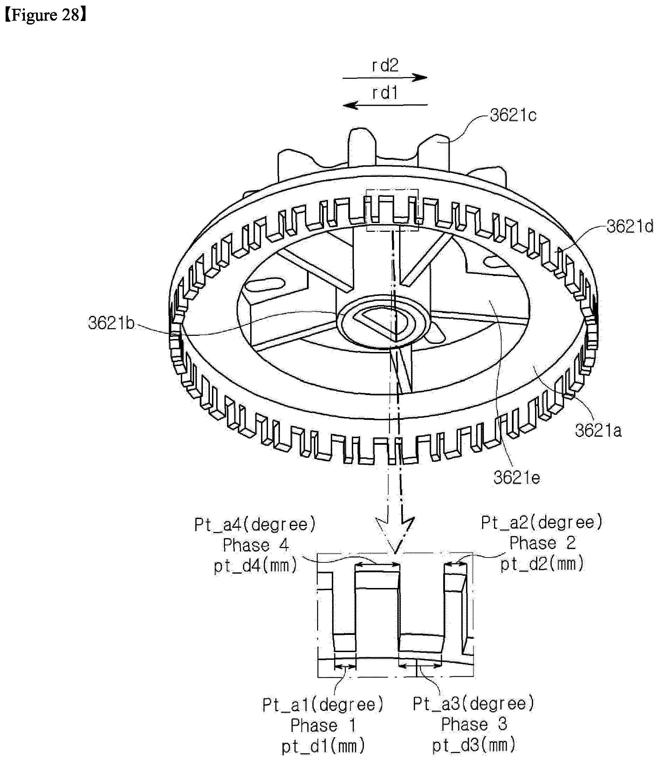

The transmission gear 514 and the drive gear 3621 will be described in detail with reference to FIGS. 27 and 28 together with FIG. 24.

FIG. 27 shows an enlarged drive gear 3621 and an enlarge transmission gear 514 according to the embodiment of the present disclosure. FIG. 28 is a perspective view of the drive gear 3621 as viewed from the bottom according to the embodiment of the present disclosure.

The transmission gear 514 may be connected to the rotation shaft member 5151 of the compression rotating portion 515. The gear teeth 5142 which meshes with the drive gear 3621 are disposed on the outer periphery of the lower portion of the transmission gear 514. A gear shaft 5141 which is coaxially connected to the rotation shaft member 5151 is disposed at the upper center of the transmission gear 514.

The gear shaft 5141 may be inserted into the receiving space of the collector body 511 through a hole formed in the bottom cover 517. The gear shaft 5141 may be configured to be inserted into a hollow formed in the rotation shaft member 5151. That is, the diameter of the outer peripheral surface of the gear shaft 5141 may be smaller than the diameter of the outer peripheral surface of the rotation shaft member 5151.

The gear shaft 5141 and the rotation shaft member 5151 may have a mechanical structure that causes them to mesh each other so as to rotate at the same angular velocity. For example, the mechanical structure may be a protrusion and groove structure.

The drive gear 3621 may include a gear body 3621a, a shaft connector 3621b and the gear teeth 3621c.

The gear body 3621a forms the exterior of the drive gear 3621. The gear teeth 3621c which meshes with the gear teeth 5142 of the transmission gear 514 is formed on the gear body 3621a. A pattern 3621d in which protrusions and grooves alternate continuously may be formed on the lower circumference of the gear body 3621a.

The upper diameter of the gear body 3621a on which the gear teeth 3621c are disposed and the lower diameter of the gear body 3621a on which the pattern 3621d is formed may be different from each other.

The shaft connector 3621b connected to the motor shaft of the compression motor 3622 is formed at the center of the gear body 3621a. The shaft connector 3621b may be formed in a cylindrical shape within the gear body 3621a. The shaft connector 3621b may have a hole having a shape corresponding to the motor shaft such that the motor shaft is inserted thereinto.

Accordingly, when the compression motor 3622 rotates, the drive gear 3621 rotates together.

A plurality of ribs 3621e may be radially disposed on the outer circumferential surface of the shaft connector 3621b in order to support the shape of the shaft connector 3621b.

The pattern 3621d is formed on the drive gear 3621 such that the widths of the protrusions adjacent to each other are different from each other and the widths of the grooves adjacent to each other are different from each other. The protrusion and groove formed in the pattern 3621d may be defined as a phase which is distinguished according to the size of the formed width.

Here, an order in which the phases of the pattern 3621d are arranged may vary according to a first direction d1 with respect to the circumference of the gear body 3621a and a second direction d2 opposite to the first direction d1.

The pattern 3621d may be composed of a set of four phases. Here, a first phase and a third phase may be formed in the form of a protrusion, and a second phase and a fourth phase may be formed in the form of a groove. In other words, the projections and the grooves may be alternately formed.

Here, the widths of the first to fourth phases may be formed to be different from each other, and each phase may be distinguished by the size of the width. Taking the embodiment shown in FIG. 28 as an example, a protrusion having a width of pt_d1 mm may be defined as the first phase, a groove having a width of pt_d2 mm may be defined as the second phase, a protrusion having a width of pt_d3 mm may be defined as the third phase, and a groove having a width of pt_d4 mm may be defined as the fourth phase.

The arrangement order of each phase may be repeated in the order of 1-2-3-4 when the gear body 3621a of the drive gear 3621 is viewed while moving in a first rotation direction rd1. Accordingly, when the gear body 3621a of the drive gear 3621 is viewed while moving in a second rotation direction rd2 opposite to the first rotation direction rd1, each phase is repeated in the order of 4-3-2-1.

As such, through the directional arrangement of the pattern 3621d formed on the drive gear 3621, a controller 700 to be described later can detect whether the drive gear 3621 is currently rotating in the first rotation direction rd1 or in the second rotation direction rd2.

FIG. 29 is a perspective view showing a state where the first collector 510 is inserted into the chamber body 361 according to the embodiment of the present disclosure. FIG. 30 is a view showing a flow path of air related to dust collection in the state where the first collector 510 is inserted into the chamber body 361 according to the embodiment of the present disclosure.

Large dust is separated while the air introduced into the first dust receiving space S1 of the collector body 511 through the first flow path 381 passes through the mesh net 5121. The air without the large dust is introduced into the second dust receiving space S2.

Then, even fine dust is separated while the air introduced into the second dust receiving space S2 passes through the cyclone 513. The air without the fine dust is introduced into the exhaust flow path 518 provided in the collector body 511.

The air which has exited the exhaust flow path 518 flows to the dust collection motor 391 via the pre-filter module 470.

Hereinafter, a detailed structure of the second collector 520 will be described.

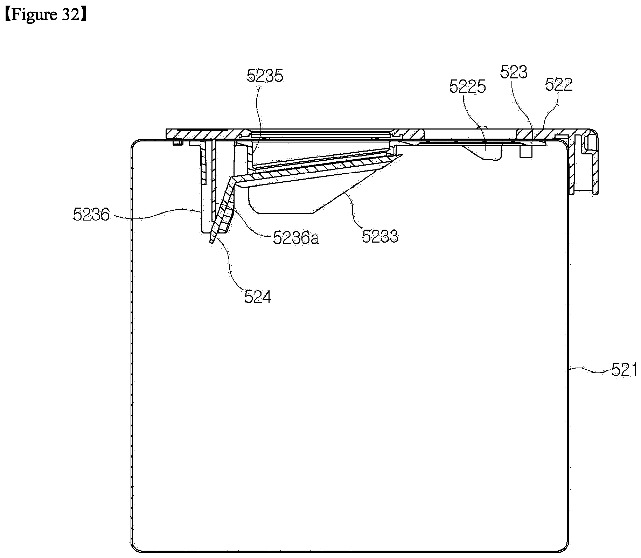

FIG. 31 is a view showing that components related to the second collector 520 are separated and deployed according to the embodiment of the present disclosure. FIG. 32 is a cross-sectional view of the second collector 520 as viewed from the side thereof according to the embodiment of the present disclosure

Referring to FIGS. 31 and 32, the second collection unit 520 may include the dust bag 521, the outer plate 522, and an inner plate 523.

The dust bag 521 is configured to receive the sucked dust from the cleaner 200 and store it therein.

When the suction force is generated by the dust collection motor 391, the volume of the dust bag 521 may be increased and the dust may be received within the dust bag. To this end, the dust bag 521 may be made of a breathable material. More specifically, the dust bag 521 may be made of a material that transmits air but does not transmit foreign substances such as dust. For example, the dust bag 521 may be made of a non-woven fabric material and may have a hexahedral shape based on an increase in volume.

A dust inlet 5212 is formed in the dust bag 521. The dust inlet 5212 is formed in the top of the dust bag 521 to pass through the dust bag 521, and is a path that guides the air and dust introduced from the air inlet 3611 of the chamber body 361 to the inside of the dust bag 521.

The dust bag 521 may include a light transmission hole 5211. The light transmission hole 5211 may be formed to pass through the dust bag 521. The light transmission hole 5211 may be formed in a position facing one surface of a transmissive panel 524 to be described later. That is, the light transmission hole 5211 may be disposed in the front of the dust inlet 5212. In the state where the second collector 520 is completely inserted into the collector receiving space 360a, the sterilization module 450 may be disposed above the light transmission hole 5211. Accordingly, the sterilization light can be irradiated into the dust bag 521 through the transmissive panel 524.

The outer plate 522 may be coupled to the outside top of the dust bag 521.

The outer plate 522 may include a quadrangular flat plate body 5221.

A portion of left and right ends of the plate body 5221 may be inserted into the sliding space of the rail body 3631. From another point of view, the left and right ends of the plate body 5221 may be fitted and coupled to the rail body 3631, and one surface of the plate body 5221 may be supported by an inner bottom surface of the rail body 3631. Through this configuration, the plate body 5221 can be inserted into the rail body 3631 in a sliding manner.

A dust inlet 5222 is formed in the plate body 5221. The dust inlet 5222 is a path that guides the air and dust introduced from the air inlet 3611 of the chamber body 361 to the inside of the dust bag 521.

The outer plate 522 may include a handle 5223.

The handle 5223 may be coupled to the front end of the plate body 5221. The handle 5223 may be integrally formed with the plate body 5221. The handle 5223 may be vertically connected to the plate body 5221.

The user may grip the handle 5223 to push the outer plate 522 into the rail body 3631. The user may grip the handle 5223 to take out the outer plate 522 from the rail body 3631.

The outer plate 522 may include a connection member 5224.

The connection member 5224 may be disposed in the rear of the dust inlet 5222. The connection member 5224 may be formed to protrude from the bottom surface of the outer plate 522 by a predetermined length. The connection member 5224 may pass through the dust bag 521 and be coupled to the inner plate 523 to be described later.

The outer plate 522 may include a support member 5225.

The support member 5225 may be disposed in the front of the outer plate 522. The support member 5225 may be formed to protrude from the bottom surface of the outer plate 522 by a predetermined length.

The support members 5225 are formed on the left and right sides of the outer plate 522 in the form of a rib, respectively. Based on the state where the outer plate 522 is inserted into the rail body 3631, the lower end of the support member 5225 may be in contact with the lower inner surface of the rail body 3631.

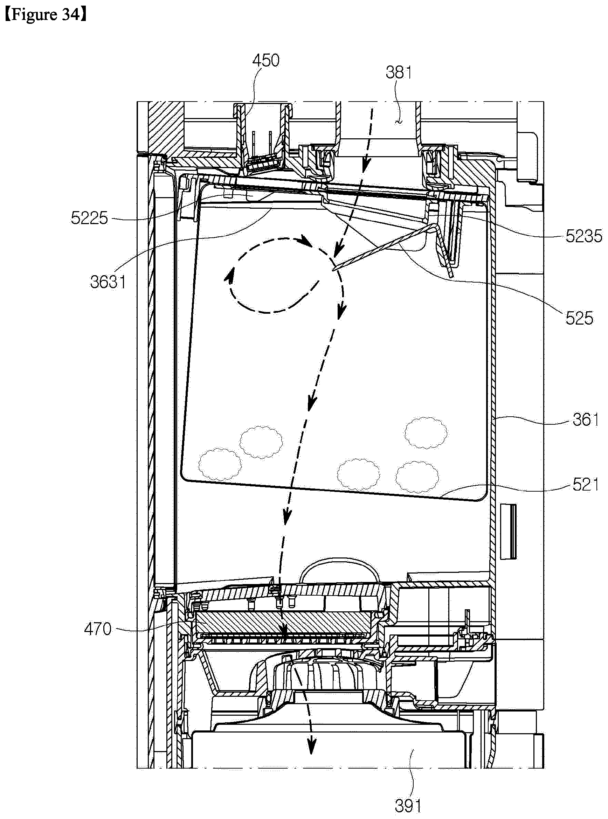

As the second collector 520 is inserted into the collector receiving space 360a, the front is lifted up by the support member 5225. That is, in the state where the second collector 520 is completely inserted into the collector receiving space 360a, the second collector 520 is inclined rearward and downward (see FIG. 34).

Through this configuration, the outer plate 522 may be supported in the lifted state toward the air inlet 3611 of the chamber body 361.

As mentioned above, the open cross-section of the air inlet 3611 in the embodiment of the present disclosure has a rearward-downward inclination. Here, since the second collector 520 is also inserted into the rail portion 363 in a state of being inclined rearward and downward, the air inlet 3611 and the dust inlet 5222 have an inclination in the same direction, so that they can be sealed without being spaced apart from each other.

The outer plate 522 may include a light transmission hole 5226.

The light transmission hole 5226 may be formed to pass through the plate body 5221. The light transmission hole 5226 may be formed in a position facing one surface of the transmissive panel 524 to be described later. That is, the light transmission hole 5226 may be disposed in the front of the dust inlet 5222.

In the state where the second collector 520 is completely inserted into the collector receiving space 360a, the sterilization module 450 may be disposed above the light transmission hole 5226. Accordingly, the sterilization light can be irradiated into the dust bag 521 through the transmissive panel 524.

The inner plate 523 may be coupled to the inside top of the dust bag 521.

The inner plate 523 may include a quadrangular flat plate body 5231.

A dust inlet 5232 is formed in the plate body 5231. The dust inlet 5232 is a path that guides the air and dust introduced from the air inlet 3611 of the chamber body 361 to the inside of the dust bag 521.

The inner plate 523 may include a sidewall 5233.

The sidewall 5233 is configured to limit and guide the flow direction of the air introduced into the dust bag 521. The sidewall 5233 may be disposed adjacent to the dust inlet 5232. The sidewall 5233 may be formed to protrude downward from the inner surface of the plate body 5231. That is, the sidewall 5233 may be disposed to extend toward an interior space of the dust bag 521.

The sidewall 5233 may be symmetrically disposed on the left and right sides of the dust inlet 5232. Accordingly, the sidewall 5233 can block the air introduced into the dust inlet 5232 from flowing to the left or right.

Meanwhile, in the state where the below-described inlet cover 525 is opened, the rear flow of the air can also be blocked.

As a result, the air introduced into the dust inlet 5232 flows forward and downward.

Through this configuration, the introduced dust can be effectively exposed to the sterilization light irradiated to the front of the dust inlet 5232.

The inner plate 523 may include a connection member insertion groove 5234.

The connection member insertion groove 5234 may be disposed behind the dust inlet 5232. The connection member insertion groove 5234 may be formed in a form through which the plate body 5231 is passed. The connection member 5224 of the outer plate 522 may be inserted into the connection member insertion groove 5234.

The inner plate 523 may include an inlet tube 5235.

The inlet tube 5235 may be coupled to the lower surface of the plate body 5231. The inlet tube 5235 may be disposed to surround the dust inlet 5232. The inlet tube 5235 may be integrally formed with the plate body 5231. The open cross-section of the inlet tube 5235 may have a rearward-downward inclination.

The lower end of the inlet tube 5235 can be closed by the inlet cover 525 to be described later. Thus, the dust inlet 5232 can also be closed.

The inner plate 523 may include an inlet cover fixing member 5236.

The inlet cover fixing member 5236 may be disposed behind the dust inlet 5232. The inlet cover fixing member 5236 may be coupled to the lower surface of the plate body 5231 in a form surrounding the connection member insertion groove 5234. The inlet cover fixing member 5236 may be integrally formed with the plate body 5231.

An inlet cover insertion groove 5236a may be formed in the inlet cover fixing member 5236. At least a portion of the inlet cover 525 may be inserted and fixed in the inlet cover insertion groove 5236a. The inlet cover insertion groove 5236a has a rearward and downward inclined surface and may extend in the left and right. Accordingly, the inlet cover 525 can be inserted from the front upper portion rearward and downward.

A transmissive panel 524 may be coupled to the inner plate 523.

When the second collector 520 is inserted into the chamber body 361, the transmissive panel 524 is disposed at a position corresponding to the position at which the sterilization module 450 is disposed. That is, when the second collection unit 520 is inserted into the chamber body 361, the sterilization module 450 and the transmissive panel 524 are disposed to face each other.

The transmissive panel 524 is made of a material through which the sterilization light emitted from the sterilization module 450 can transmit toward the inside of the collector body 511. For example, the transmissive panel 524 may be made of a poly methyl methacrylate (PMMA) material.

The inlet cover 525 for opening and closing the dust inlet 5232 may be coupled to the inside of the inner plate 523. The inlet cover 525 may be disposed at an end of the inlet tube 5235.

The inlet cover 525 may be made of a material having elasticity. For example, the inlet cover 525 may be made of a rubber material.

The inlet cover 525 may open one side of the inlet tube 5235 by the suction force of the dust collection motor 391. That is, the inlet cover 525 may be opened toward the interior space of the dust bag 521.

One side of the inlet cover 525 may be inserted into and coupled to the inlet cover insertion groove 5236a.

While the inlet cover insertion groove 5236a has a large rearward-downward inclination, the open cross-section of the inlet tube 5235 has a smaller rearward-downward inclination than that of the inlet cover insertion groove 5236a. When the inlet cover 525 is inserted into the inlet cover insertion groove 5236a, the shape of the inlet cover 525 is changed in such a manner as to be bent at one point thereof.

Here, a restoring force is applied to the inlet cover 525 in a direction intended to unfold again based on the bent point.

Through this configuration, in the state the inlet cover 525 is inserted into the inlet cover insertion groove 5236a, the inlet cover 525 may be in close contact with the inlet tube 5235. That is, the inlet cover 525 does not deflect due to its own weight when the dust collection motor 391 is not driven.

The inlet cover 525 may maintain the dust inlet 5232 to be closed when the dust collection motor 391 is not driven. When the dust collection motor 391 starts to run, the inlet cover 525 may open the dust inlet 5232.

When the dust collection motor 391 is operated, the inlet cover 525 opens the inlet tube 5235 while being deformed toward the interior space of the dust bag 521, and when the dust collection motor 391 is stopped, the inlet cover 525 is restored by elasticity and closes the inlet tube 5235.

Accordingly, when the second collector 520 is separated from the cleaner station, it is possible to prevent the dust from scattering, winged insects, etc., from escaping, or odors from spreading.

In addition, when the dust collection motor 391 is not driven even in the state where the second collector 520 is coupled to the cleaner station, there is an effect preventing the dust or odor from flowing backward through the first flow path 381 because the inlet cover 525 is closing the inlet tube 5235.

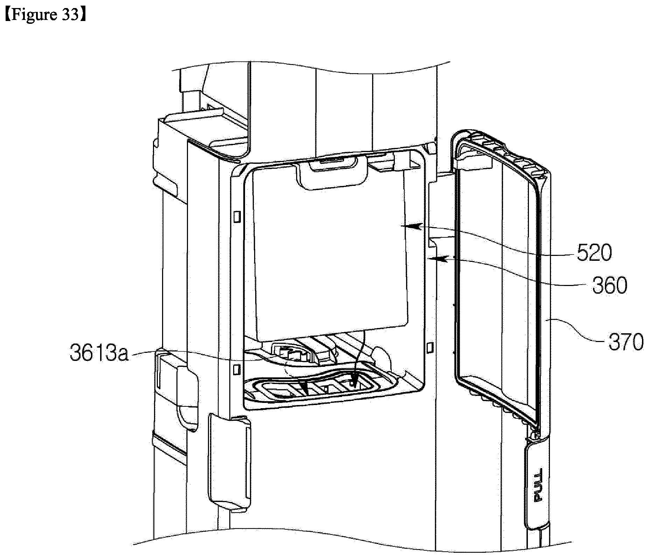

FIG. 33 is a perspective view showing a state where the second collector 520 is inserted into the chamber body 361 according to the embodiment of the present disclosure. FIG. 34 is a view showing a flow path of air related to dust collection in the state where the second collector 520 is inserted into the chamber body 361 according to the embodiment of the present disclosure.

When an airflow is generated by the suction force of the dust collection motor 391, the air which contains foreign substances and flows from the inside of the dust bin 220 of the cleaner 200 moves to the dust bag 521 through the first flow path 381, and leaves only foreign substances in the dust bag 521 and exits the dust bag 521.

Here, the dust introduced through the first flow path 381 is blocked from flowing right and left by the sidewall 5233 and is blocked from flowing in the rear direction by the inlet cover 525. Therefore, as indicated by arrows shown in FIG. 34, the dust flows forward and downward toward the sterilization light irradiated by the sterilization module 450.

In addition, the air exiting the dust bag 521 flows to the dust collection motor 391 via the pre-filter module 470.

On the other hand, when the dust collection motor 391 is driven to form an air flow in the second collector 520, the gear passing hole 3613a is exposed as it is without being blocked, so that the suction force is also applied to the gear passing hole 3613a. (see the direction of the dotted arrows in FIG. 34)

As such, when the suction force is directly applied to the compression motor 3622 through the gear passing hole 3613a, a failure of the compression motor 3622 may be caused.

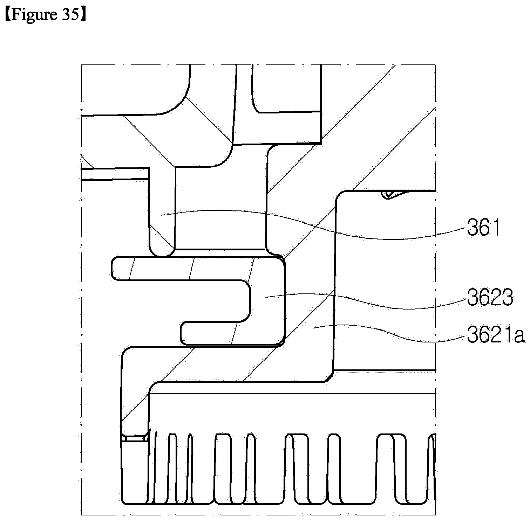

FIG. 35 is a cross-sectional view showing an enlarged sealing member 3623 that seals the gear passing hole 2623a according to the embodiment of the present disclosure.

In the embodiment according to the present disclosure, the compression driver 362 may further include the sealing member 3623 disposed between the drive gear 3621 and the chamber body 361.

The sealing member 3623 may be disposed along the entire outer circumference of the gear body 3621a of the drive gear 3621. The sealing member 3623 may be disposed between the outer surface of the gear body 3621a of the drive gear 3621 and the lower outer surface of the chamber body 361.

As such, the sealing member 3623 may seal between the gear passing hole 3613a and the compression motor 3622, thereby serving to block the air flow between the compression motor 3622 and the collector receiving space 360a.

The sealing member 3623 may include a first sealing portion that comes into contact with the chamber body 361 and a second sealing portion that comes into contact with the gear body 3621a. The first sealing portion and the second sealing portion may be connected to each other at one side end and may be spaced apart from the each other at the other side end.

Through this configuration, as the suction force is applied by the dust collection motor 391, the sealing portion is compressed in a direction in which the other side of the first sealing portion and the other side of the second sealing portion are closer to each other. Accordingly, a sealing force is further strengthened by the driving of the dust collection motor 391, and the suction force is prevented from being directly applied to the compression motor 3622.

Meanwhile, the cleaner station may further include a controller 700.

The control unit 700 is a component that controls the operation of each component of the cleaner station. The controller 700 may be mounted on a printed circuit board.

The controller 700 may include all types of devices capable of processing data, such as a processor. Here, the "processor" may refer to, for example, a data processing device embedded in hardware, which has a physically structured circuit in order to perform functions represented by codes or commands included in a program. An example of such a data processing device embedded in hardware may include a microprocessor, a central processing unit (CPU), a processor core, a multiprocessor, an application-specific integrated circuit (ASIC), a field programmable gate array (FPGA). However, the scope of the present disclosure is not limited thereto.

In the embodiment of the present disclosure, the controller 700 may detect a change in the rotation direction of the compression motor 3622.

As described above, if a force applied to the compression motor 3622 is greater than or equal to a set value when the compression motor 3622 rotates in one direction, the rotation direction of the compression motor 3622 is changed to the other direction. Here, the force applied to the compression motor 3622 is a resistance force (torque) generated by that the rotating plate 5153 compresses the dust. When the resistance force reaches a set value, the rotation direction of the compression motor 3622 is changed.

That is, only when the compression motor 3622 is connected to the rotating plate 5153 and the rotating plate 5153 compresses the dust, the rotation direction of the compression motor 3622 is changed.

The controller 700 may control the driving of the compression motor 3622 in any stage in the dust removal process.

In a possible embodiment, the driving of the compression motor 3622 may be performed at the same time that the cleaner 200 is coupled to the coupling portion 320. Information related to the coupling of the cleaner 200 may be detected by the coupling sensor 325 disposed on one side of the coupling portion 320 and be transmitted to the controller 700.

In another possible embodiment, the driving of the compression motor 3622 may be performed after the driving of the dust collection motor 391 is completed.

In further another possible embodiment, the driving of the compression motor 3622 may be performed before and after the driving of the dust collection motor 391, respectively.

The controller 700 may detect whether the rotation direction of the compression motor 3622 is changed within a predetermined period of time from a point of time when the compression motor 3622 starts to run. The predetermined period of time should be set sufficiently larger than an expected period of time from when the rotating plate 5153 starts to rotate at an initial position to when the rotating plate 5153 comes into contact with one surface of the fixed plate 5152.

The detection of a change in the rotation direction may be performed by detecting the arrangement order of the patterns 3621d formed on the drive gear 3621. To this end, the compression driver 362 may further include a photo interrupter (not shown) disposed adjacent to the drive gear 3621.

If the phases of the pattern 3621d detected by the photo interrupter have the arrangement order of 1-2-3-4, the compression motor 3622 is rotating in the first direction. If the phases of the pattern 3621d detected by the photo interrupter have the arrangement order of 4-3-2-1, the compression motor 3622 is rotating in the second direction (see FIG. 28).

The controller 700 can drive the compression motor 3622 irrespective of the type of the collector 500 currently coupled to the cleaner station.

If the first collector 510 is inserted into the chamber body 361, the rotation direction of the compression motor 3622 is necessarily changed within the predetermined period of time.

If the second collector 520 is inserted into the chamber body 361, no load is applied to the compression motor 3622 even though the predetermined period of time elapses. Therefore, the compression motor 3622 rotates only in the same direction and the rotation direction is not changed.

That is, the controller 700 drives rotationally the compression motor 3622 and determines the type of the collector 500 currently coupled to the cleaner station 300 on the basis of whether the rotation direction of the compression motor 3622 is changed within the predetermined period of time.

In the foregoing, an exemplary embodiment of the present disclosure has been illustrated and described. However, the present invention is not limited to the described specific embodiment. Various modifications can be made by those skilled in the art without departing from the subject matter of the present invention as defined by the appended claims. Also, these modifications should not be understood individually from the spirit or perspective of the present invention.

According to the present disclosure, the user can use either the first collector formed in the form of a bin having the fixed-sized dust receiving space or the second collector in the form of a bag having the variably-sized dust receiving space in combination with the cleaner station. Therefore, there is an advantage that it is possible to satisfy various preferences of users who prefer different types of collectors.

Advantageous effects of the present disclosure are not limited to the above-described effects and other unmentioned effects can be clearly understood from the detailed description of the present disclosure by those skilled in the art to which the present disclosure belongs.

**REFERENCE NUMERALS**

| | | | |
|---|---|---|---|
| 3: | Cleaner System | | |
| 200: | Cleaner | | |
| 220: | Dust B | | |
| 300: | Cleaner Station | | |
| 310: | Housing | | |
| 360: | Chamber | | |
| 360a: | Collector Receiving Space | 361: | Chamber Body |
| 3611: | Air Inlet | 3612: | Air Outlet |
| 3613: | Drive Gear Receiving Portion | 3613a: | Gear Passing Hole |
| 362: | Compression Driver | 3621: | Drive Gear |
| 3622: | Compression Motor | 3623: | Sealing Member |
| 363: | Rail Portion | 3631: | Rail Body |
| 3632: | Interference Protrusion | 3633: | Protrusion Guide |
| 364: | Sterilization Module Mounting Portion | | |
| 365: | Filter Mounting Portion | | |
| 366: | Filter Detection Protrusion | 3661: | Protrusion Body |
| 3662: | Catching Protrusion | | |
| 370: | Housing Cover | | |
| 371: | Cover Protrusion | | |
| 391: | Dust Collection Motor | | |
| 450: | Sterilization Module | | |
| 470: | Prefilter Module | | |
| 471, 472: | Filter Case | 4712: | Pressing Protrusion |
| 473, 474: | Filter | | |
| 500: | Collector | | |
| 510: First | Collector | | |
| 511: | Collector Body | 515: | Compression Rotating Portion |
| 516: | Body Cover | 5161: | First Cover Portion |
| 5161c: | Rib | 5162: | Second Cover Portion |
| 520: | Second Collector | | |
| 521: | Dust Bag | 522: | Outer Plate |
| 5225: | Support Member | 523: | Inner Plate |
| 700: | Controller | | |

## Claims

1. A cleaner station comprising:
a housing which is coupled to a dust bin of a cleaner;
a dust collection motor which is disposed within the housing and generates a suction force sucking dust within the dust bin;
a collector that is provided with a dust receiving space which collects the dust sucked from the inside of the dust bin by the dust collection motor; and
a chamber which is disposed in the housing and forms a collector receiving space such that the collector is separably coupled to the chamber,
wherein the collector that is coupled to the chamber comprises a first collector which is formed in the form of a bin having the fixed-sized dust receiving space and comprises a cyclone as a dust separation means and a second collector which comprises the variably-sized dust receiving space and a breathable material-made dust bag as the dust separation means, and the first and second collectors are compatible,
and wherein the chamber comprises:
a chamber body which forms an exterior of the collector receiving space; and
a rail portion which is disposed on an upper portion of the chamber body, allows at least a portion of the second collector to be fitted and coupled thereto, and supports the second collector such that a bottom surface of the chamber body is spaced apart from the second collector by a predetermined distance.

2. The cleaner station of claim 1, wherein the collector receiving space has a cross-sectional area of a front end thereof larger than a cross-sectional area of a rear end thereof.

3. The cleaner station of claim 1,
wherein the front of the chamber body is opened or closed by a housing cover rotatably coupled to one side of the housing,
and wherein the rail portion comprises:
a rail body which forms a space in which the second collector slides;
an interference protrusion which is disposed at a first position that interferes with the housing cover in front of the rail body and moves to a second position that avoids interference with the housing cover by contact with the first collector or the second collector; and
a protrusion guide which is coupled to the interference protrusion and guides movement of the interference protrusion.

4. The cleaner station of claim 1,
wherein the chamber further comprises:
a filter mounting portion which is disposed in a lower portion of the chamber body and to which a pre-filter module that filters air which has passed through the collector is coupled in an attachable and detachable manner; and
a filter detection protrusion of which one side is pressed by the pre-filter module and which is moved in a direction to avoid interference with the first collector.

5. The cleaner station of claim 4,
wherein the pre-filter module comprises:
a filter case;
a filter which is received within the filter case; and
a pressing protrusion which is formed to protrude from one side of the filter case.

6. The cleaner station of claim 5,
wherein the filter detection protrusion comprises:
a protrusion body; and
a catching protrusion which is formed to protrude from an outer surface of the protrusion body and comes into contact with the pressing protrusion as the pre-filter module is coupled to the filter mounting portion.

7. The cleaner station of claim 1, wherein the chamber further comprises a compression driver which is disposed in a lower portion of the chamber body and generates power for rotating a compression rotating portion that compresses the dust collected in the first collector.

8. The cleaner station of claim 7,
wherein the first collector comprises a transmission gear which is coupled to the compression rotating portion and transmits the power from the compression driver to the compression rotating portion,
wherein the compression driver comprises:
a compression motor which is disposed outside the collector receiving space; and
a drive gear which is connected to a shaft of the compression motor outside the collector receiving space and is meshed with the transmission gear,
and wherein a gear passing hole which exposes at least a portion of the drive gear toward an inside of the collector receiving space is formed in the lower portion of the chamber body such that the drive gear and the transmission gear are meshed with each other.

9. The cleaner station of claim 8, wherein the compression driver further comprises a sealing member disposed between the drive gear and the chamber body in order to seal the gear passing hole.

10. The cleaner station of claim 8, further comprising a controller which detects a change in a rotation direction of the compression motor.

11. The cleaner station of claim 10, wherein the controller determines a type of the collector coupled to the chamber on the basis of whether the change in a rotation direction of the compression motor is detected within a predetermined period of time.

12. The cleaner station of claim 7,
wherein the chamber further comprises:
an air inlet which is provided in a top of the chamber body and has an open cross-section having a rearward-downward inclination; and
a sterilization module mounting portion which is disposed to be spaced apart from the air inlet and is equipped with a sterilization module that is provided to irradiate ultraviolet light toward the collector receiving space.

13. The cleaner station of claim 12,
wherein the first collector comprises:
a collector body which forms an exterior of the dust receiving space;
a transmission gear which is disposed outside and below the collector body, is coupled to the compression rotating portion, and transmits the power from the compression driver to the compression rotating portion; and
a body cover which covers an open top of the collector body,
and wherein a rib which is inserted into the rail portion is formed to protrude from the body cover.

14. The cleaner station of claim 13,
wherein a rearward and downward inclined surface is formed in some areas of a top surface of the body cover,
and wherein a dust inlet positioned to face the air inlet is formed in the inclined surface, on the basis of a state where the first collector is inserted into the chamber body.

15. The cleaner station of claim 12,
wherein the second collector comprises:
an outer plate which is coupled to an outside top of the dust bag, is fitted and coupled to the rail portion, is inserted into the collector receiving space in a sliding manner, and comprises a dust inlet formed therein; and
a support member which is formed to protrude from a bottom surface of the outer plate, has a lower portion coming into contact with one surface of the rail portion, and supports a state where the outer plate is lifted toward the air inlet.
